# EUROPEAN PATENT APPLICATION

(11) **EP 4 205 765 A2**
(43) Date of publication of application: **05.07.2023**
(21) Application number: 21862153.0
(22) Date of filing: 30.08.2021
(51) Int. Cl.: A61K 39/395, A61K 9/08, A61K 47/26, A61K 47/18, A61K 47/20, A61K 47/22, A61K 47/12, A61K 9/00, C07K 16/28

(54) **STABLE PHARMACEUTICAL PREPARATION**

(30) Priority: 31.08.2020 KR 20200110695
(71) Applicant: Celltrion, Inc., Incheon 22014 (KR)
(72) Inventor: ROH, Ji Won, Incheon 22014 (KR); KIM, Kwang Woo, Incheon 22014 (KR); KIM, Su Jung, Incheon 22014 (KR); SHIN, Yeon Kyeong, Incheon 22014 (KR); HAN, Won Yong, Incheon 22014 (KR); OH, Jun Seok, Incheon 22014 (KR); LEE, Jae Bin, Incheon 22014 (KR)
(74) Representative: Cabinet Laurent & Charras
(86) International application number: PCT/KR2021/011626
(87) International publication number: WO 2022/045857

(57) **Abstract**

The stable pharmaceutical formulation according to the present disclosure comprises (A) an antibody or antigen-binding fragment thereof that binds to an interleukin-6 receptor; (B) a surfactant; (C) stabilizers; and (D) a buffer. The stable pharmaceutical formulation according to the present disclosure has a low viscosity even when it contains an antibody, especially at a high concentration, and has excellent long-term storage stability based on excellent stability under accelerated and severe conditions, and may be administered intravenously or subcutaneously.

## Description

### CROSS-REFERENCE TO RELATED APPLICATION(S)

This application claims priority to Korean Patent Application No. 10-2020-0110695, filed on August 31, 2020, the disclosure of which is incorporated herein by reference in its entirety.

### BACKGROUND

### Technical Field

The present disclosure relates to a stable pharmaceutical formulation comprising an antibody that binds to interleukin-6 receptor (IL-6R).

### Description of the Related Art

Interleukin-6 (IL-6) is one of cytokines involved in acute inflammatory response regulation, B cell differentiation, T cell activation, bone metabolism, thrombosis, immune response, and the like. Interleukin 6 (IL-6) has been implicated in various autoimmune diseases, inflammatory diseases, malignancies, etc. Such abnormal disease may be treated by using an antibody that binds to interleukin-6 receptor (IL-6R).

Antibody refers to an immunoglobulin molecule consisting of four polypeptide chains in which two heavy chains and two light chains are linked to each other by disulfide bonds. Each heavy chain consists of a heavy chain variable region and a heavy chain constant region. The heavy chain constant region consists of three domains (CH1, CH2, and CH3). Each light chain consists of a light chain variable region and a light chain constant region. The light chain constant region consists of one domain (CL). The heavy chain variable region and the light chain variable region may be further subdivided into a hypervariable region called a complementarity determining region (CDR), which is arranged together with a more conserved region called a framework region (FR). Each of the heavy and light chain variable regions consists of three CDRs and four FRs, which are arranged in the following order from the amino terminus to the carboxy terminus: FR1, CDR1, FR2, CDR2, FR3, CDR3, FR4.

Antibodies that bind to the interleukin-6 receptor are unstable proteins. These antibodies are susceptible to physical or chemical changes due to heat stress, light stress, or the like. Thus, stability and/or activity may be compromised after the product has been stored, especially after storage for a long period of time.

That is, there is a problem in that it is difficult to formulate a pharmaceutical composition including a physiologically active protein such as an antibody while ensuring stability so that protein activity can be maintained for an appropriate time.

### SUMMARY

The problem to be solved by the present disclosure is to provide a stable pharmaceutical formulation, including an antibody that binds to an interleukin-6 receptor.

Another problem to be solved by the present disclosure is to provide a vial filled with the pharmaceutical formulation.

Another problem to be solved by the present disclosure is to provide a cartridge filled with the pharmaceutical formulation.

Another problem to be solved by the present disclosure is to provide a pre-filled syringe, filled with the pharmaceutical formulation.

Another problem to be solved by the present disclosure is to provide an auto-injector in which the pre-filled syringe is included.

As a result of repeated research to overcome the above-described problems, the present inventors have developed a stable formulation comprising an antibody that binds to the interleukin-6 receptor.

The present disclosure provides a stable pharmaceutical formulation containing: (A) an antibody or antigen-binding fragment thereof that binds to an interleukin-6 receptor; (B) a surfactant; (C) a stabilizer which is i) an amino acid or an amino acid derivative, ii) a sugar or a sugar alcohol or a mixture thereof; and (D) a buffer, wherein the antibody or antigen-binding fragment thereof comprises a light chain variable region comprising a CDR1 domain comprising the amino acid sequence of SEQ ID NO: 1, a CDR2 domain comprising the amino acid sequence of SEQ ID NO: 2, and a CDR3 domain comprising amino acids of SEQ ID NO: 3; and a heavy chain variable region comprising a CDR1 domain comprising the amino acid sequence of SEQ ID NO: 4, a CDR2 domain comprising the amino acid sequence of SEQ ID NO: 5, and a CDR3 domain comprising the amino acid sequence of SEQ ID NO: 6.

In one embodiment of the present disclosure, the pharmaceutical formulation may be in a liquid form, but is not limited thereto.

### DESCRIPTION OF SPECIFIC EMBODIMENTS

### (A) Antibody or antigen-binding fragment thereof

In the present disclosure, the term "antibody" refers to an immunoglobulin molecule consisting of four polypeptide chains in which two heavy chains and two light chains are linked to each other by disulfide bonds, and may comprise naturally occurring antibodies having other changed structures, such as camelid antibodies.

In one embodiment of the present disclosure, the antibody or antigen-binding fragment thereof according to the present disclosure is a polyclonal antibody, a monoclonal antibody, a recombinant antibody, a single-chain antibody, a hybrid antibody, a chimeric antibody, a humanized antibody or fragment thereof (antigen-binding fragment), preferably a monoclonal antibody or fragment thereof.

In the present disclosure, the antibody or antigen-binding fragment thereof according to the present disclosure may be a humanized monoclonal antibody or a fragment thereof, most preferably a humanized immunoglobulin (Immunoglobulin G, IgG) monoclonal antibody, and may be prepared by a known method.

In the present disclosure, the antigen-binding fragment comprises any naturally occurring, enzymatically obtainable, synthetic or genetically engineered polypeptide or glycoprotein that specifically binds to an antigen to form a complex, and for example, there is a nanobody and the like.

In the present disclosure, the humanized antibody is also called a reshaped human antibody, and may be one wherein the complementarity determining region (CDR) of a non-human mammal, for example, a mouse antibody is grafted into the complementarity determining region of a human antibody, and may be prepared by other commonly known genetic recombination techniques.

In the present disclosure, the antibody or antigen-binding fragment thereof may comprise a light chain variable region comprising a CDR1 domain comprising the amino acid sequence of SEQ ID NO: 1, a CDR2 domain comprising the amino acid sequence of SEQ ID NO: 2, and a CDR3 domain of the amino acid sequence of SEQ ID NO: 3; and a heavy chain variable region comprising a CDR1 domain comprising the amino acid sequence of SEQ ID NO: 4, a CDR2 domain comprising the amino acid sequence of SEQ ID NO: 5, and a CDR3 domain comprising the amino acid sequence of SEQ ID NO: 6.

In the present disclosure, the antibody or antigen-binding fragment thereof comprises a light chain variable region comprising the amino acid sequence of SEQ ID NO: 7; and a heavy chain variable region comprising the amino acid sequence of SEQ ID NO: 8.

In the present disclosure, the antibody or antigen-binding fragment thereof comprises a light chain comprising the amino acid sequence of SEQ ID NO: 9; and a heavy chain comprising the amino acid sequence of SEQ ID NO: 10.

In the present disclosure, the antibody or antigen-binding fragment thereof according to the present disclosure may be tocilizumab, sarilumab, sapelizumab, vobarilizumab, or a mixture thereof. Preferably, the antibody or antigen-binding fragment thereof may be tocilizumab, sapelizumab, vobarilizumab or a mixture thereof. Most preferably, the antibody or antigen-binding fragment thereof may be tocilizumab.

In the present disclosure, the 'tocilizumab' may be hPM-1 described in International Publication No. WO1992-019759, the original drug substance as well known in the art, or one biosimilar thereto.

In the present disclosure, the 'sarilumab' may be an interleukin-6 receptor binding antibody, also known as KEVZARA^{®}, or one biosimilar thereto.

In the present disclosure, the 'sapelizumab' may be an interleukin-6 receptor-binding antibody, also known as SA-237 or satralizumab, or one biosimilar thereto.

In the present disclosure, the 'vobarilizumab', also known as ALX-0061, may be an interleukin-6 receptor binding nanobody linked to an anti-human serum albumin nanobody or one biosimilar thereto.

In one embodiment of the present disclosure, the concentration of the antibody or antigen-binding fragment thereof may be 1 to 300 mg/ml, preferably 20 to 250 mg/ml, more preferably 50 to 220 mg/ml, most preferably 100 to 200 mg/ml. When the concentration of the antibody or antigen-binding fragment thereof is within the above-mentioned range, the degree of freedom of administration dose and administration cycle may be increased, and long-term stability and low viscosity (1 cP to 15 cP, preferably 1 cP to 10 cP) are excellent.

In another embodiment of the present disclosure, (A) the antibody or antigen-binding fragment thereof may be included in a high concentration of 50 mg/ml or more. For example, a formulation containing a monoclonal antibody (mAb) drug at a high concentration has different stability and colloidal properties from the formulation at a low protein concentration due to the complexity of the molecule itself and various intermolecular interactions, and it makes a development of a stable pharmaceutical formulation difficult since the higher the level of a concentration of the protein, the more aggregation and degradation are promoted. In addition, reversible self-binding occurs, which affects an increase of viscosity, etc., and causes difficulties in developing a stable formulation and manufacturing a medical product. The present inventors have confirmed through experiments that the formulation of the present disclosure is stable at a high concentration despite the above-mentioned difficulties.

### (B) Surfactant

In the present disclosure, the term "surfactant" refers to a substance which may be used to significantly increase the water solubility of a hydrophobic or oily substance or increase the miscibility of two substances having different hydrophobic properties.

In the present disclosure, the surfactant according to the present disclosure may be polyoxyethylene sorbitan fatty acid ester (e.g., polysorbate, etc.), polyoxyethylene alkyl ether (e.g., Brij^{®}, etc.), alkylphenylpolyoxyethylene ether (e.g., Triton-X, etc.), polyoxyethylene-polyoxypropylene copolymer (e.g., poloxamer, Pluronic^{®}, etc.), sodium dodecyl sulfate (SDS), etc., but the present disclosure is not limited thereto.

In the present disclosure, the surfactant may be polyoxyethylene sorbitan fatty acid ester, polyoxyethylene alkyl ether, alkylphenylpolyoxyethylene ether, polyoxyethylene-polyoxypropylene copolymer, sodium dodecyl sulfate, or a mixture thereof. The surfactant may be preferably a polyoxyethylene sorbitan fatty acid ester, a polyoxyethylene-polyoxypropylene copolymer, or a mixture thereof, more preferably a polyoxyethylene sorbitan fatty acid ester.

In the present disclosure, the surfactant may be polysorbate, poloxamer, or a mixture thereof, preferably polysorbate.

In the present disclosure, the surfactant may be polysorbate 20, polysorbate 40, polysorbate 60, polysorbate 80, or a mixture thereof, preferably polysorbate 80.

In one embodiment of the present disclosure, the concentration of the surfactant may be freely adjusted within a range that does not adversely affect the stability and viscosity of the stable pharmaceutical formulation according to the present disclosure.

In one embodiment of the present disclosure, (B) the concentration of surfactant may be 0.001 to 1% (w/v), preferably 0.005 to 0.1% (w/v), most preferably 0.01 to 0.05% (w/v). When the concentration of the surfactant is within the above-mentioned range, it exhibits excellent long-term stability and low viscosity.

### (C) Stabilizer

In the present disclosure, the term "stabilizer" is a substance that is physiologically acceptable and imparts stability to the formulation.

In one embodiment of the present disclosure, (C) the stabilizer may be i) an amino acid or an amino acid derivative; ii) sugars or sugar alcohols; or a mixture thereof.

In one embodiment of the present disclosure, i) the amino acid or amino acid derivative (provided that it is different from histidine contained in the buffer according to the present disclosure) may be any one or more selected from the group consisting of threonine, methionine, arginine, proline, leucine, glycine, taurine, phenylalanine, tryptophan, glutamine, aspartate, glutamate, alanine, asparagine, serine, glycine, and tyrosine, but is not limited thereto. In the present disclosure, i) the amino acid or amino acid derivative may be preferably any one or more selected from the group consisting of threonine, methionine, arginine, proline, leucine, glycine, and taurine.

In one embodiment of the present disclosure, ii) the sugar or sugar alcohol may be any one or more selected from the group consisting of sucrose, trehalose, sorbitol, glucose, fructose, galactose, xylose, maltose, lactose, xylitol, mannitol, D-maltitol, inositol, lactitol and isomalt, but is not limited thereto. In the present disclosure, ii) the sugar or sugar alcohol may be preferably any one or more selected from the group consisting of sucrose, trehalose, and sorbitol.

In one embodiment of the present disclosure, the stabilizer may be preferably a mixture of threonine and methionine.

In the present disclosure, a concentration ratio of the mixture of threonine and methionine (i.e., threonine: methionine) may be 1:1 to 10:1, preferably 1:1 to 8:1, more preferably 1:1 to 7:1.

In the present disclosure, a concentration of the stabilizer may be freely adjusted within a range that does not adversely affect the stability and viscosity of the pharmaceutical formulation according to the present disclosure.

In one embodiment of the present disclosure, a concentration of the amino acid or amino acid derivative may be 10 to 500 mM, preferably 30 to 450 mM, more preferably 100 to 400 mM, and most preferably 130 to 300 mM.

In one embodiment of the present disclosure, a concentration of threonine may be 5 to 300 mM, preferably 100 to 250 mM, more preferably 110 to 190 mM.

In one embodiment of the present disclosure, a concentration of methionine may be 5 to 200 mM, preferably 10 to 150 mM, more preferably 30 to 110 mM.

In one embodiment of the present disclosure, a concentration of the sugar or sugar alcohol may be 0.1 to 30% (w/v), preferably 5 to 10% (w/v).

In the present disclosure, when the concentration of the stabilizer according to the present disclosure is within the range described in the present disclosure, long-term stability and low viscosity are excellent.

### (D) Buffer

In the present disclosure, the term "buffer" is a substance that minimizes the change in pH caused by acid or alkali.

In one embodiment of the present disclosure, (D) the buffer may be histidine or a salt thereof, acetic acid or a salt thereof, phosphoric acid or a salt thereof, citric acid or a salt thereof, succinic acid or a salt thereof, glutamic acid or a salt thereof, 2-(N-morpholino) ethanesulfonic acid (MES) or a salt thereof, tromethamine (Tris) or a salt thereof, etc., but the present disclosure is not limited thereto. Preferably, the buffer may be histidine or a salt thereof, acetic acid or a salt thereof, phosphoric acid or a salt thereof, citric acid or a salt thereof, succinic acid or a salt thereof or a mixture thereof.

In the present disclosure, the buffer may be more preferably histidine or a salt thereof or a mixture thereof.

For example, the histidine salt according to the present disclosure may be histidine hydrochloride, histidine acetate, histidine phosphate, histidine sulfate, etc., but is not limited thereto.

For example, the acetate salt according to the present disclosure may be sodium acetate, zinc acetate, aluminum acetate, ammonium acetate, potassium acetate, and the like, but is not limited thereto.

For example, the phosphate salt according to the present disclosure may be potassium phosphate, sodium phosphate, ammonium phosphate, calcium phosphate, magnesium phosphate, etc., but is not limited thereto.

For example, the citrate according to the present disclosure may be sodium citrate, calcium citrate, potassium citrate, or the like, but is not limited thereto.

For example, the succinate according to the present disclosure may be sodium succinate, calcium succinate, potassium succinate, sodium sulfosuccinate, potassium sulfosuccinate, calcium sulfosuccinate, etc., but is not limited thereto.

For example, the glutamate according to the present disclosure may be sodium glutamate, potassium glutamate, ammonium glutamate, or the like, but is not limited thereto.

For example, the 2-(N-morpholino) ethanesulfonic acid (MES) salt according to the present disclosure may be MES chloride, MES sodium, etc., but is not limited thereto.

For example, the tris salt according to the present disclosure may be tris hydrogen chloride, tris acetate, tris borate, and the like, but is not limited thereto.

In the present disclosure, a content of the buffer may be freely adjusted within a range that does not substantially adversely affect the stability and viscosity of the pharmaceutical formulation according to the present disclosure.

In one embodiment of the present disclosure, (D) the content of the buffer may be 0.1 to 50 mM, preferably 1 to 25 mM.

### (E) pH

In the present disclosure, the pH of the stable pharmaceutical formulation according to the present disclosure can be adjusted in a range that optimizes the therapeutic efficacy by using a buffer or a pH adjusting agent.

In the present disclosure, the pH may be 5 or more and less than 7. When the pH is within the above-mentioned range, it exhibits excellent in long-term stability and low viscosity.

In the present disclosure, the term "pH adjusting agent" is a substance used to adjust the pH of the formulation.

In one embodiment of the present disclosure, the pharmaceutical formulation according to the present disclosure may further include a pH adjusting agent (acid or base). The content of the pH adjusting agent may be freely adjusted within a range that does not substantially adversely affect the stability and viscosity of the formulation.

In the present disclosure, the pH adjusting agent may be acetic acid, phosphoric acid, sodium hydroxide, sodium hydrogen carbonate, and the like, but is not limited thereto.

### (F) Other ingredients

In the present disclosure, the stable pharmaceutical formulation according to the present disclosure may not contain a preservative. The preservative may be a preservative commonly used in the relevant art. For example, the preservative may be octadecyldimethylbenzyl ammonium chloride, hexamethonium chloride, benzalkonium chloride, benzethonium chloride, phenol, butyl alcohol, benzyl alcohol, alkyl paraben, catechol, resorcinol, cyclohexanol, 3-pentanol, m-cresol, or the like, but is not limited thereto. Preferably, the formulation does not contain a preservative, so that stability may be improved.

As used herein, the term "not including" or "does not include" means that it does not contain any component at all or does not contain any component substantially, that is, it includes the component in a range that does not affect the activity of the antibody, stability or viscosity of the pharmaceutical formulation. For example, any component based on the total weight of the pharmaceutical formulation means being included with 1% (w/v) or less, 1 mM or less, 1 mg/ml or less, 1 ppm (w/v) or less, or 1 ppb (w/v) or less.

In the present disclosure, the stable pharmaceutical formulation of the present disclosure may further include additives known in the art within a range that does not substantially adversely affect the stability and viscosity of the formulation. The additive may be, for example, an aqueous carrier, an antioxidant, or a mixture thereof. In the present disclosure, the aqueous carrier may be a carrier useful for the preparation of a pharmaceutical formulation that is safe and non-toxic when administered to humans. Examples of the aqueous carrier include, but are not limited to, sterile water for injection (SWFI), bacteristatic water for injection (BWFI), sterile saline solution, Ringer's solution, dextrose, and the like. In the present disclosure, the antioxidant includes, but is not limited to, ascorbic acid.

### (G) "Stable" pharmaceutical formulation

In the present disclosure, the term "stable" or "stabilization" means that during the manufacturing process and/or during store or storage, the component according to the present disclosure or a composition or formulation containing the same exhibits its physical stability, chemical stability and/or biological activity. In the present disclosure, various analytical techniques for measuring stability are readily available in the art.

In the present disclosure, the physical stability may be evaluated by a method known in the art, for example, it may be evaluated by measuring the sample apparent attenuation of light (absorption or optical density). This measurement of light attenuation is related to the turbidity of the formulation. In addition, the high molecular weight of component content, the low molecular weight of component content, the amount of intact protein, and the number of insoluble foreign matter particles may be measured for physical stability.

In the present disclosure, the chemical stability may be evaluated by a method known in the art, for example, by detecting and quantifying the chemically changed form of the antibody to measure charge change that may be evaluated by ion exchange chromatography (e.g., a change in charge that occurs as a result of deamidation or oxidation) or a charge variant (measurement of an acidic or basic peak), etc.

In the present disclosure, the biological activity may be evaluated by a method known in the art, for example, by measuring the antigen binding affinity through enzyme-linked immunosorbent assay (ELISA).

In one embodiment of the present disclosure, the term "stable" pharmaceutical formulation means a pharmaceutical formulation satisfying one or more of the following (G)-1 to (G)-11.

### (G)-1 Turbidity

- A pharmaceutical formulation wherein an absorbance A₆₀₀ is 0 to 0.03, as measured with a spectrophotometer, after storage for 0 day, 5 days, 10 days, 2 weeks, 3 weeks or 4 weeks at a temperature of 5±3°C;
- A pharmaceutical formulation wherein an absorbance A₆₀₀ is 0 to 0.06, as measured with a spectrophotometer, after storage for 0 day, 5 days, 10 days, 2 weeks, 3 weeks, 4 weeks or 6 weeks at a temperature of 40±2°C, a relative humidity of 75±5%, and a closed condition;

### (G)-2 main component content (Main peak%)

- A pharmaceutical formulation wherein the main component is 98% to 100% as measured by SEC-HPLC, after storage for 0 day, 5 days, 10 days, 2 weeks, 3 weeks or 4 weeks at a temperature of 5±3°C;
- A pharmaceutical formulation wherein the main component is 97 % to 100% as measured by SEC-HPLC, after storage for 0 day, 5 days, 10 days, 2 weeks, 3 weeks, 4 weeks or 6 weeks at a temperature of 40±2°C, a relative humidity of 75±5% and a closed condition;

### (G)-3 High molecular weight of component content (a peak (pre-peak%) whose retention time is earlier than that of the main peak (intact IgG))

- A pharmaceutical formulation wherein a high molecular weight of component content is 0 to 1.5% as measured by SEC-HPLC, after storage for 0 day (0 week), 5 days, 10 days, 2 weeks, 3 weeks or 4 weeks at a temperature of 5±3°C;
- A pharmaceutical formulation wherein a high molecular weight of component content is 0 to 2% as measured by SEC-HPLC, after storage for 0 week, 5 days, 10 days, 2 weeks, 3 weeks, 4 weeks or 6 weeks at a temperature of 40±2°C, a relative humidity of 75±5% and a closed condition;

### (G)-4 Low molecular weight of component content (a peak (post peak%) whose retention time is later than that of the main peak (intact IgG))

- A pharmaceutical formulation wherein a low molecular weight of component content is 0 to 1% as measured by SEC-HPLC, after storage for 0 day (0 week), 5 days, 10 days, 2 weeks, 3 weeks or 4 weeks at a temperature of 5±3°C;
- A pharmaceutical formulation wherein a low molecular weight of component content is 0 to 1.5% as measured by SEC-HPLC, after storage for 0 week, 5 days, 10 days, 2 weeks, 3 weeks, 4 weeks or 6 weeks at a temperature of 40±2°C, a relative humidity of 75±5% and a closed condition;

### (G)-5 Intact immunoglobulin G content

- A pharmaceutical formulation wherein content of intact immunoglobulin G (Intact IgG%) is 96% to 100% as measured by non-reducing CE-SDS after storage for 0 day (0 week), 5 days, 10 days, 2 weeks, 3 weeks or 4 weeks at a temperature of 5±3°C;
- A pharmaceutical formulation wherein content of intact immunoglobulin G (Intact IgG%) is 93% to 100% as measured by non-reducing CE-SDS, after storage for 0 week, 5 days, 10 days, 2 weeks, 3 weeks, 4 weeks or 6 weeks at a temperature of 40±2°C, a relative humidity of 75±5% and a closed condition;

### (G)-6 Intact heavy chain and light chain content

- A pharmaceutical formulation wherein content of intact heavy chain and light chain (Intact HC+ LC%) is 99% to 100% as measured by reducing CE-SDS, after storage for 0 day (0 week), 5 days, 10 days, 2 weeks, 3 weeks or 4 weeks at a temperature of 5±3°C;
- A pharmaceutical formulation wherein content of intact heavy chain and light chain (Intact HC+ LC%) is 98% to 100% as measured by reducing CE-SDS, after storage for 0 week, 5 days, 10 days, 2 weeks, 3 weeks, 4 weeks or 6 weeks at a temperature of 40±2°C, a relative humidity of 75±5% and a closed condition;

### (G)-7 Number of insoluble foreign matter particles

- A pharmaceutical formulation wherein the number of insoluble foreign matter particles (10 µm≤, <100 µm) measured by MFI is 0 to 1,000 and the number of insoluble foreign matter particles (25 µm≤, <100 µm) is 0 to 150, after storage for 0 day (0 week), 5 days, 10 days, 2 weeks, 3 weeks or 4 weeks at a temperature of 5±3°C;
- A pharmaceutical formulation wherein the number of insoluble foreign matter particles (10 µm≤, <100 µm) measured by MFI is 0 to 2,000 and the number of insoluble foreign matter particles (25 µm≤, <100 µm) is 0 to 200, after storage for 0 week, 5 days, 10 days, 2 weeks, 3 weeks, 4 weeks or 6 weeks at a temperature of 40± 2°C, a relative humidity of 75 ± 5%, and a closed condition;

### (G)-8 Oxidation rate

- A pharmaceutical formulation wherein an oxidation rate of Met 106 of heavy chain is 0 to 6% as measured by LC-MS, after storage for 0 day (0 week), 5 days, 10 days, 2 weeks, 3 weeks or 4 weeks at a temperature of 5±3°C;
- A pharmaceutical formulation wherein an oxidation rate of Met 106 of heavy chain is 0% to 10% as measured by LC-MS, after storage for 0 week, 5 days, 10 days, 2 weeks, 3 weeks, 4 weeks or 6 weeks at a temperature of 40±2°C, a relative humidity of 75±5% and a closed condition;

### (G)-9 Charge variant (main peak excluding acidic or basic peaks in ion exchange chromatography)

- A pharmaceutical formulation wherein a main peak is 60 to 70% as measured by IEC-HPLC, after storage for 0 day (0 week), 5 days, 10 days, 2 weeks, 3 weeks or 4 weeks at a temperature of 5±3°C;
- A pharmaceutical formulation wherein a main peak is 40% to 60% as measured by IEC-HPLC, after storage for 0 week, 5 days, 10 days, 2 weeks, 3 weeks, 4 weeks or 6 weeks at a temperature of 40±2°C, a relative humidity of 75±5% and a closed condition;

### (G)-10 IL-6R binding affinity

- A pharmaceutical formulation wherein an IL-6R binding affinity is 80% to 120% as measured by ELISA, after storage for 0 week, 5 days, 10 days, 2 weeks, 3 weeks, 4 weeks or 6 weeks at a temperature of 40±2°C, a relative humidity of 75±5% and a closed condition;

### (G)-11 Viscosity

- A pharmaceutical formulation wherein a viscosity is 1 cP to 11 cP as measured by viscometer, after storage for 0 day (0 week), 5 days, 10 days, 2 weeks, 3 weeks or 4 weeks at a temperature of 5±3°C;
- A pharmaceutical formulation wherein a viscosity is 1 cP to 15 cP as measured by viscometer, after storage for 0 week, 5 days, 10 days, 2 weeks, 3 weeks, 4 weeks or 6 weeks at a temperature of 40±2°C, a relative humidity of 75±5% and a closed condition.

In one embodiment of the present disclosure, the viscosity of the stable pharmaceutical formulation according to the present disclosure may be 1 to 15 cP, preferably 1 to 10 cP.

In another embodiment of the present disclosure, the stable pharmaceutical formulation according to the present disclosure contains:

(A) 1 to 300 mg/ml of an antibody or antigen-binding fragment thereof comprising a light chain variable region comprising a CDR1 domain comprising the amino acid sequence of SEQ ID NO: 1, a CDR2 domain comprising the amino acid sequence of SEQ ID NO: 2, and a CDR3 domain comprising the amino acid sequence of SEQ ID NO: 3; and a heavy chain variable region comprising a CDR1 domain comprising the amino acid sequence of SEQ ID NO: 4, a CDR2 domain comprising the amino acid sequence of SEQ ID NO: 5, and a CDR3 domain comprising the amino acid sequence of SEQ ID NO: 6; (B) 0.001 to 1% (w/v) of a surfactant; (C) 10 to 500 mM of an amino acid or an amino acid derivative as a stabilizer; and (D) 0.1 to 50 mM of a buffer.

In another embodiment of the present disclosure, the stable pharmaceutical formulation according to the present disclosure contains:

(A) 1 to 300 mg/ml of an antibody or antigen-binding fragment thereof comprising a light chain variable region comprising a CDR1 domain comprising the amino acid sequence of SEQ ID NO: 1, a CDR2 domain comprising the amino acid sequence of SEQ ID NO: 2, and a CDR3 domain comprising the amino acid sequence of SEQ ID NO: 3; and a heavy chain variable region comprising a CDR1 domain comprising the amino acid sequence of SEQ ID NO: 4, a CDR2 domain comprising the amino acid sequence of SEQ ID NO: 5, and a CDR3 domain comprising the amino acid sequence of SEQ ID NO: 6; (B) 0.001 to 1% (w/v) of a surfactant; (C) 0.1 to 30% (w/v) of a sugar or sugar alcohol as a stabilizer; and (D) 0.1 to 50 mM of a buffer.

### [Product]

In one embodiment of the present disclosure, the present disclosure provides a stable pharmaceutical formulation according to the present disclosure; and a product comprising a container receiving the stable pharmaceutical formulation in a closed state.

The stable pharmaceutical formulation is as described above herein.

In the present disclosure, the container may be formed of a material such as glass, polymer (e.g., plastic), metal, etc., but is not limited thereto.

For example, the container according to the present disclosure may be a bottle, a vial, a cartridge, a syringe (e.g., a pre-filled syringe), an auto-injector, etc., or a tube, preferably a glass or polymer vial, or a glass or polymer pre-filled syringe, but the present disclosure is not limited thereto.

In one embodiment of the present disclosure, the present disclosure provides a vial filled with the pharmaceutical formulation according to the present disclosure, a cartridge filled with the pharmaceutical formulation, and a pre-filled syringe filled with the pharmaceutical formulation, or auto-injector in which pre-filled syringe is contained.

Specific product types such as the vial, cartridge, pre-filled syringe, auto-inj ector, etc., and the method of filling the stable pharmaceutical formulation into the vial, cartridge, pre-filled syringe, auto-injector, etc. may be easily obtained or carried out by one skilled in the technical field to which the present disclosure belongs. For example, U.S. Patent Nos. 4,861,335 and 6,331,174 etc., disclose specific product types of pre-filled syringes and methods of filling thereto. For example, U.S. Patent Nos. 5,085,642, 5,681,291 and the like disclose specific product types of auto-injectors and its assembly methods. Also, as the vial, cartridge, pre-filled syringe, auto-injector, etc., a commercially available product may be used as it is, or a separately custom-made product may be used in consideration of the physical properties, administration site, and dosage of the stable pharmaceutical formulation.

In the present disclosure, the container may be a container for a single administration.

In the present disclosure, the product according to the present disclosure may further comprise instructions for providing the method of use, storage, or both of the stable pharmaceutical formulation. The instructions may comprise a treatment method, administration route, dosage, or administration timing for interleukin-6 receptor-related diseases.

In the present disclosure, the product may comprise other tools necessary from a commercial and user point of view, for example, a needle, a syringe, and the like.

### [Method for preparing stable pharmaceutical formulation]

The stable pharmaceutical formulation of the present disclosure can be prepared by a known method, and is not limited to a specific preparation method. For example, after adjusting the pH while adding a buffer to a solution containing a surfactant and a stabilizer, an antibody may be added to the mixed solution to prepare a pharmaceutical formulation. As another example, after preparing a solution including the antibody, buffer and stabilizer in the final step of the purification process, a surfactant may be added to the solution to prepare a pharmaceutical formulation.

### [How to use stable pharmaceutical formulation]

In the present disclosure, the stable pharmaceutical formulation according to the present disclosure may exhibit a therapeutic effect on diseases associated with interleukin-6 receptor.

In the present disclosure, diseases associated with interleukin-6 receptor may be an autoimmune disease, an inflammatory disease, a malignant tumor, Still's disease, vasculitis, juvenile idiopathic arthritis, osteoarthritis, and the like. For example, diseases associated with the interleukin-6 receptor are rheumatoid arthritis (RA), adult onset still's disease (AOSD), systemic juvenile idiopathic arthritis (sJIA), polyarticular juvenile idiopathic arthritis (pJIA), castleman's disease (CD), giant cell arteritis (GCA), Takayasu's arteritis (TAK), systemic sclerosis (SSc), systemic sclerosis-associated interstitial lung disease (SSc-ILD), cytokine release syndrome (CRS), hand osteoarthritis, polymyalgia rheumatica(PMR), antineutrophil cytoplasmic antibody associated vasculitis (ANCA-AAV), relapsing polychondritis (RP), type 2 diabetes (T2D), ankylosing spondylitis (AS), axial spondyloarthritis (axSpA), psoriasis (Ps), psoriatic arthritis (PsA), inflammatory bowel disease (IBD), Crohn's disease (CD), ulcerative colitis (UC), thyroid associated ophthalmopathy (TAO), rheumatoid arthritis-associated cardiovascular disease, acute graft versus host disease (GVHD), non-ST-segment elevation MI (myocardial infarction) (NSTEMI), systemic lupus erythematosus (SLE), schizophrenia, uveitis, ovarian cancer, neuromyelitis optica (NMO), glomerulonephritis (GN), chronic glomerulonephritis, colorectal cancer, pneumonia, lung cancer, etc., but the present disclosure is not limited thereto. Preferably, diseases associated with interleukin-6 receptor may be any one or more selected from the group consisting of rheumatoid arthritis, adult onset still's disease, systemic juvenile idiopathic arthritis, polyarticular juvenile idiopathic arthritis, Castleman's disease, giant cell arteritis, Takayasu's arteritis, systemic sclerosis, systemic sclerosis-associated interstitial lung disease, cytokine release syndrome, and polymyalgia rheumatica.

In the present disclosure, the stable pharmaceutical formulation according to the present disclosure may be used for intravenous (IV) or subcutaneous (SC) administration.

In one embodiment of the present disclosure, the stable pharmaceutical formulation may be subjected to a dilution step before use. In the present disclosure, the subcutaneous (SC) formulation of the present disclosure may be used as an intravenous (IV) formulation through a dilution step.

In one embodiment of the present disclosure, the stable pharmaceutical formulation may not be subject to reconstitution step before use.

The concentration of other components as well as the antibody in the pharmaceutical formulation is as described above, and the total volume of the pharmaceutical formulation according to the present disclosure may be 0.9 to 20 mL

The dosage and timing of administration of the pharmaceutical formulation may vary depending on the type of disease, the severity and course of the disease, the patient's health and response to treatment, and the judgment of the treating doctor, and are not limited to the specific dosage and timing of administration.

### [Treatment method and stabilization method]

In one embodiment of the present disclosure, the present disclosure provides a method for treating diseases associated with an interleukin-6 receptor, including: administering a stable pharmaceutical formulation comprising (A) an antibody or antigen-binding fragment thereof that binds to the interleukin-6 receptor; (B) a surfactant, (C) a stabilizer, and (D) a buffer to a patient having diseases associated with an interleukin-6 receptor.

In another embodiment of the present disclosure, the present disclosure provides a method for stabilizing an antibody in the pharmaceutical formulation, comprising preparing a stable pharmaceutical formulation containing (A) an antibody or antigen-binding fragment thereof that binds to the interleukin-6 receptor; (B) a surfactant, (C) a stabilizer, and (D) a buffer.

The stable pharmaceutical formulation is as described above herein.

The treatment method or stabilization method according to the present disclosure is pursuant to the above description herein.

Each of the features described herein may be used in combination, and the fact that each of the features is recited in different dependent claims of the claims does not indicate that they cannot be used in combination.

### Advantageous Effects

The stable pharmaceutical formulation according to the present disclosure has a low viscosity even when it contains an antibody, especially at a high concentration, and has excellent long-term storage stability based on excellent stability under accelerated and severe conditions, and may be administered intravenously or subcutaneously.

### BRIEF DESCRIPTION OF THE DRAWINGS

FIG. 1 is a measurement result of a main component content of Examples 1 to 5.
FIG. 2 is a measurement result of a high molecular weight of component content of Examples 1 to 5.
FIG. 3 is a measurement result of a low molecular weight of component content of Examples 1 to 5.
FIG. 4 is a measurement result of a main component content of Examples 6 to 13.
FIG. 5 is a measurement result of a high molecular weight of component content of Examples 6 to 13.
FIG. 6 is a measurement result of a low molecular weight of component content of Examples 6 to 13.
FIG. 7 is a measurement result of a main component content of Examples 14 to 16.
FIG. 8 is a measurement result of a high molecular weight of component content of Examples 14 to 16.
FIG. 9 is a measurement result of a low molecular weight of component content of Examples 14 to 16.
FIG. 10 is a measurement result of a main component content of Examples 17 to 22.
FIG. 11 is a measurement result of a high molecular weight of component content of Examples 17 to 22.
FIG. 12 is a measurement result of a low molecular weight of component content of Examples 17 to 22.
FIG. 13 is a measurement result of a main component content of Examples 23 to 27.
FIG. 14 is a measurement result of a high molecular weight of component content of Examples 23 to 27.
FIG. 15 is a measurement result of a low molecular weight of component content of Examples 23 to 27.
FIG. 16 is a measurement result of a main component content of Examples 25, and 28 to 30.
FIG. 17 is a measurement result of a high molecular weight of component content of Examples 25, and 28 to 30.
FIG. 18 is a measurement result of a low molecular weight of component content of Examples 25, and 28 to 30.
FIG. 19 is a measurement result of the charge variant of Examples 31and 32 and Comparison Example 1.
FIG. 20 is a measurement result of the IL-6R binding affinity of Examples 33 to 35, and Comparison Example 2.

### DESCRIPTION OF SPECIFIC EMBODIMENTS

Hereinafter, the present disclosure will be described in detail by Examples. The following Examples are only illustrative of the present disclosure, and do not limit the scope of present disclosure in any way. The documents cited in the present disclosure are incorporated herein by reference.

With respect to the antibody used in the following Experimental Examples, tocilizumab cultured and purified at Celltrion Research Institute was used. The following method was used as a method for measuring the physical stability, chemical stability, and biological activity of a pharmaceutical formulation to be described later.

### - Turbidity

The absorbance at 600 nm was measured using a UV-Vis spectrophotometer.

### - Main component content

The main component content (main peak; %) was measured using size exclusion high performance liquid chromatography (SEC-HPLC).

### - High molecular weight of component content

The content of high molecular of main component (pre-peak; %) was measured using Size Exclusion High Performance Liquid Chromatography (SEC-HPLC).

### - Low molecular weight of component content

The content of the low molecular weight of component (post-peak; %) was measured using Size Exclusion High Performance Liquid Chromatography (SEC-HPLC).

### - Content of intact immunoglobulin G (Intact IgG%)

The content (%) of intact immunoglobulin G was measured using non-reduced capillary electrophoresis-sodium dodecyl sulfate (NR CE-SDS).

### - Content of intact heavy chain and light chain (Intact HC+LC%)

Content (%) of intact heavy chain and light chain was measured using reduced capillary electrophoresis-sodium dodecyl sulfate (R CE-SDS).

### - Number of insoluble foreign matter particle (Sub-visible particles)

The number of insoluble foreign matter particles was measured using micro flow imaging (MFI).

### - Oxidation

The oxidation (%) of heavy chain Met 106 was measured by peptide mapping with liquid chromatography-mass spectrometry (LC-MS) through mass analysis.

### - Charge variant

Main peak (%) was measured using ion exchange chromatography-high performance liquid chromatography (IEC-HPLC).

### - IL-6R binding affinity

IL-6R binding affinity (%) was measured using enzyme-linked immunoSorbent assay (ELISA).

### - Viscosity

Using a micro-capillary rheometer (apparent shear rate range: 10³ to 10⁵ s⁻¹) equipped with a flow cell (B05 sensor type, 50 µm cell depth), the measurement was carried out in a 500 µl syringe at 25±0.1°C.

### Experimental Example 1: Preparation of pharmaceutical formulation comprising antibody binding to interleukin-6 receptor (IL-6R)

Examples 1 to 37 of Table 1 below were prepared by preparing each buffer suitable for each pH, adding a stabilizer, then adding an antibody, and then adding a surfactant. Specific content of each component is as described in Table 1 below.

**[Table 1]**

| Classification | Antibody | Surfactant | Stabilizer | Buffer | pH |
|---|---|---|---|---|---|
| **Example 1** | 180 mg/ml | Polysorbate 80 0.02 %(w/v) | Arginine 150 mM | Sodium acetate 20 mM | 5.5 |
| **Example 2** | 180 mg/ml | Polysorbate 80 0.02 %(w/v) | Arginine 150 mM | Sodium phosphate 20 mM | 6 |
| **Example 3** | 180 mg/ml | Polysorbate 80 0.02 %(w/v) | Arginine 150 mM | Sodium citrate 20 mM | 6 |
| **Example 4** | 180 mg/ml | Polysorbate 80 0.02 %(w/v) | Arginine 150 mM | Sodium succinate 20 mM | 6 |
| **Example 5** | 180 mg/ml | Polysorbate 80 0.02 %(w/v) | Arginine 150 mM | Histidine 20 mM | 5.5 |
| **Example 6** | 180 mg/ml | Polysorbate 80 0.02 %(w/v) | Sorbitol 5.0 %(w/v) | Sodium acetate 20 mM | 5.5 |
| **Example 7** | 180 mg/ml | Polysorbate 80 0.02 %(w/v) | Sucrose 10.0 %(w/v) | Sodium acetate 20 mM | 5.5 |
| **Example 8** | 180 mg/ml | Polysorbate 80 0.02 %(w/v) | Trehalose 10.0 %(w/v) | Sodium acetate 20 mM | 5.5 |
| **Example 9** | 180 mg/ml | Polysorbate 80 0.02 %(w/v) | Threonine 300 mM | Sodium acetate 20 mM | 5.5 |
| **Example 10** | 180 mg/ml | Polysorbate 80 0.02 %(w/v) | Proline 300 mM | Sodium acetate 20 mM | 5.5 |
| **Example 11** | 180 mg/ml | Polysorbate 80 0.02 %(w/v) | Arginine 100 mM | Sodium acetate 20 mM | 5.5 |
| | | | Leucine 50 mM | | |
| **Example 12** | 180 mg/ml | Polysorbate 80 0.02 %(w/v) | Taurine 300 mM | Sodium acetate 20 mM | 5.5 |
| **Example 13** | 180 mg/ml | Polysorbate 80 0.02 %(w/v) | Glycine 300 mM | Sodium acetate 20 mM | 5.5 |
| **Example 14** | 180 mg/ml | Polysorbate 80 0.02 %(w/v) | Taurine 300 mM | Histidine 20 mM | 6 |
| **Example 15** | 180 mg/ml | Polysorbate 80 0.02 %(w/v) | Threonine 300 mM | Histidine 20 mM | 6 |
| **Example 16** | 180 mg/ml | Polysorbate 80 0.02 %(w/v) | Threonine 200 mM | Histidine 20 mM | 6 |
| | | | Methionine 100 mM | | |
| **Example 17** | 180 mg/ml | Polysorbate 80 0.02 %(w/v) | Threonine 140 mM | Histidine 10 mM | 6 |
| | | | Methionine 60 mM | | |
| **Example 18** | 180 mg/ml | Polysorbate 80 0.02 %(w/v) | Threonine 180 mM | Histidine 10 mM | 6 |
| | | | Methionine 60 mM | | |
| **Example 19** | 180 mg/ml | Polysorbate 80 0.02 %(w/v) | Threonine 160 mM | Histidine 10 mM | 6 |
| | | | Methionine 40 mM | | |
| **Example 20** | 180 mg/ml | Polysorbate 80 0.02 %(w/v) | Threonine 160 mM | Histidine 10 mM | 6 |
| | | | Methionine 80 mM | | |
| **Example 21** | 180 mg/ml | Polysorbate 80 0.02 %(w/v) | Threonine 110 mM | Histidine 10 mM | 6 |
| | | | Methionine 110 mM | | |
| **Example 22** | 180 mg/ml | Polysorbate 80 0.02 %(w/v) | Threonine 189mM | Histidine 10 mM | 6 |
| | | | Methionine 31mM | | |
| **Example 23** | 180 mg/ml | Polysorbate 80 0.02 %(w/v) | Threonine 160 mM | Histidine 25 mM | 6 |
| | | | Methionine 60 mM | | |
| **Example 24** | 180 mg/ml | Polysorbate 80 0.02 %(w/v) | Threonine 160 mM | Histidine 15 mM | 6 |
| | | | Methionine 60 mM | | |
| **Example 25** | 180 mg/ml | Polysorbate 80 0.02 %(w/v) | Threonine 160 mM | Histidine 10 mM | 6 |
| | | | Methionine 60 mM | | |
| **Example 26** | 180 mg/ml | Polysorbate 80 0.02 %(w/v) | Threonine 160 mM | Histidine 5 mM | 6 |
| | | | Methionine 60 mM | | |
| **Example 27** | 180 mg/ml | Polysorbate 80 0.02 %(w/v) | Threonine 160 mM | Histidine 1mM | 6 |
| | | | Methionine 60 mM | | |
| **Example 28** | 100 mg/ml | Polysorbate 80 0.02 %(w/v) | Threonine 160 mM | Histidine 10 mM | 6 |
| | | | Methionine 60 mM | | |
| **Example 29** | 160 mg/ml | Polysorbate 80 0.02 %(w/v) | Threonine 160 mM | Histidine 10 mM | 6 |
| | | | Methionine 60 mM | | |
| **Example 30** | 200 mg/ml | Polysorbate 80 0.02 %(w/v) | Threonine 160 mM | Histidine 10 mM | 6 |
| | | | Methionine 60 mM | | |
| **Example 31** | 180 mg/ml | Polysorbate 80 0.02 %(w/v) | Threonine 160 mM | Histidine 10 mM | 5.7 |
| | | | Methionine 60 mM | | |
| **Example 32** | 180 mg/ml | Polysorbate 80 0.02 %(w/v) | Threonine 160 mM | Histidine 10 mM | 6.3 |
| | | | Methionine 60 mM | | |
| **Example 33** | 180 mg/ml | Polysorbate 80 0.01 %(w/v) | Threonine 160 mM | Histidine 10 mM | 6 |
| | | | Methionine 60 mM | | |
| **Example 34** | 180 mg/ml | Polysorbate 80 0.05 %(w/v) | Threonine 160 mM | Histidine 10 mM | 6 |
| | | | Methionine 60 mM | | |
| **Example 35** | 180 mg/ml | Poloxamer 188 0.02 %(w/v) | Threonine 160 mM | Histidine 10 mM | 6 |
| | | | Methionine 60 mM | | |
| **Compar ative Example 1** | 180 mg/ml | Polysorbate 80 0.02 %(w/v) | Threonine 160 mM | Histidine 10 mM | 7 |
| | | | Methionine 60 mM | | |
| **Compar ative Example 2** | 180 mg/ml | Polysorbate 80 0.00 %(w/v) | Threonine 160 mM | Histidine 10 mM | 6 |
| | | | Methionine 60 mM | | |

### Experimental Example 2: Comparison of buffer

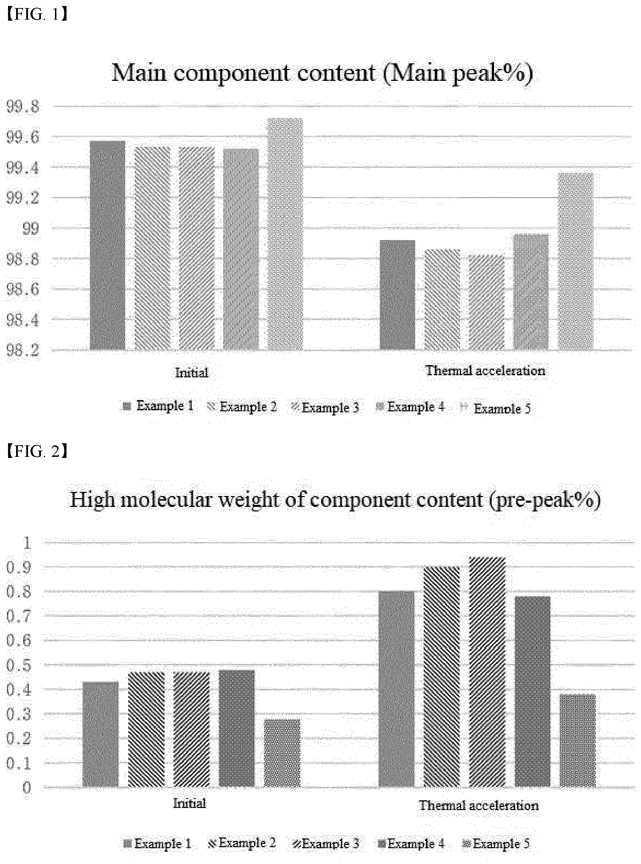

Stability of Examples 1 to 5 prepared according to Experimental Example 1 was measured under initial conditions and thermal acceleration conditions, and the results were shown in Table 2 and FIGS. 1 to 3 below. The initial condition was tested with a sample stored for 0 to 5 days at a temperature of 5±3°C, and the thermal acceleration condition was tested with a sample stored for 10 days at a temperature of 40±2°C and 75±5% relative humidity.

**[Table 2]**

| Evaluation example and result | Example 1 | Example 2 | Example 3 | Example 4 | Example 5 | |
|---|---|---|---|---|---|---|
| Buffer 20 mM | Sodium acetate | Sodium phosphate | Sodium citrate | Sodium succinate | Histidine | |
| Antibody (mg/ml) | 180 | 180 | 180 | 180 | 180 | |
| Polysorbate 80 (%(w/v)) | | 0.02 | 0.02 | 0.02 | 0.02 | 0.02 |
| Arginine (mM) | | 150 | 150 | 150 | 150 | 150 |
| Initial | Turbidity | 0.0147 | 0.0122 | 0.0104 | 0.0115 | 0.0135 |
| | Main component content (Main peak%) | 99.57 | 99.53 | 99.53 | 99.52 | 99.72 |
| | High molecular weight of component content (%) | 0.43 | 0.47 | 0.47 | 0.48 | 0.28 |
| | Low molecular weight of component content (%) | 0 | 0 | 0 | 0 | 0 |
| | Viscosity (cP) | 6.47 | 6.34 | 6.8 | 6.48 | 6.37 |
| Thermal accelerati on | Turbidity | 0.0171 | 0.0146 | 0.0179 | 0.019 | 0.0165 |
| | Main component content (Main peak%) | 98.92 | 98.86 | 98.82 | 98.96 | 99.36 |
| | High molecular weight of component content (%) | 0.8 | 0.9 | 0.94 | 0.78 | 0.38 |
| | Low molecular weight of component content (%) | 0.28 | 0.24 | 0.24 | 0.26 | 0.26 |
| | Viscosity (cP) | 6.55 | 6.23 | 7.2 | 6.5 | 6.12 |

Under initial conditions, it was found that all of Examples 1 to 5 show turbidity of 0.03 or less, main component content of 98% or more, high molecular weight of component content of 1.5% or less, low molecular weight of component content of 1% or less, viscosity of 1 cP to 11 cP, and are pharmaceutically acceptable and stable.

Among them, Examples 1 and 5 were the highest as 99.57% and 99.72%, respectively, in the main component content and was the lowest as 0.43 and 0.28, respectively, in the high molecular weight of component content, indicating that the examples including acetic acid or histidine buffer had the highest stability.

Under the thermal acceleration condition, it was found that all of Examples 1 to 5 show turbidity of 0.06 or less, main component content of 97% or more, high molecular weight of component content of 2% or less, low molecular weight of component content of 1.5% or less, viscosity of 1 cP to 15 cP, and are pharmaceutically acceptable and stable.

Among them, it was found that Example 5 was the highest as 99.36% in the main component content and was the lowest as 0.38% in the content of high molecular weight of component, indicating that the example including the histidine buffer had the highest stability.

In Experimental Example 2, the stability of the examples including the acetic acid or histidine buffer was measured as being high, and in Experimental Example 3, various examples including the acetic acid buffer were additionally tested, and in Experimental Example 4, several examples including the histidine buffer were additionally tested.

### Experimental Example 3: Comparison of stabilizers in examples including acetic acid buffer

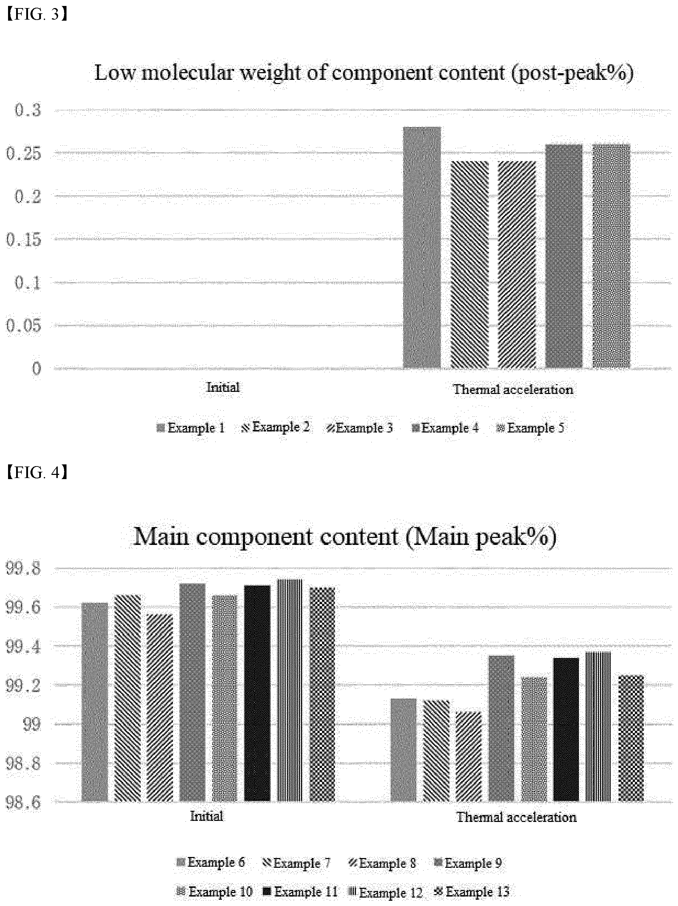

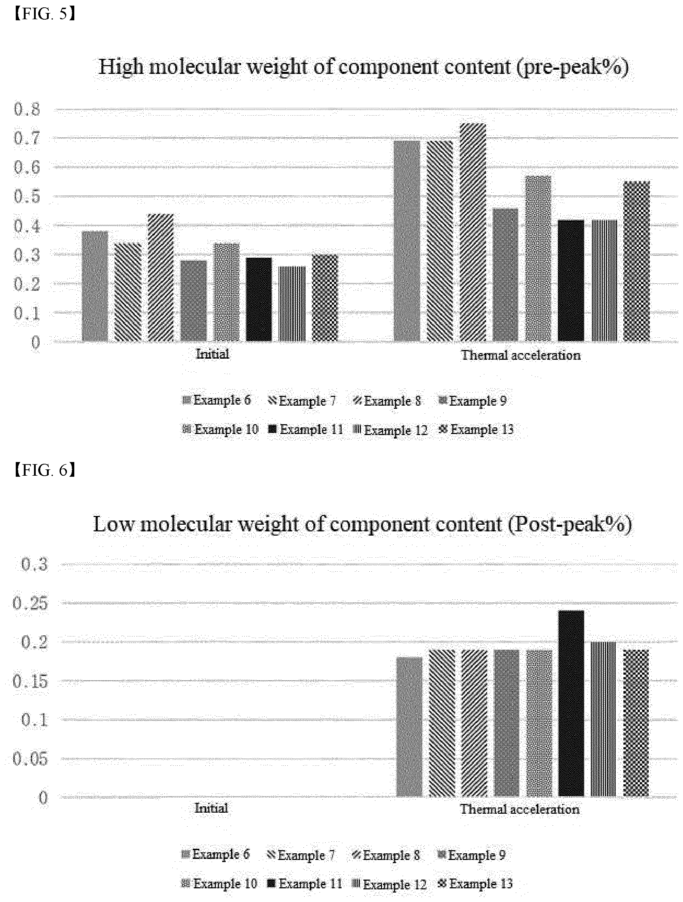

Stability of Examples 6 to 13 prepared according to Experimental Example 1 was measured under initial conditions and thermal acceleration conditions, and the results were shown in Table 3 and FIGS. 4 to 6 below. The initial condition was tested with a sample stored for 0 day at a temperature of 5±3°C, and the thermal acceleration condition was tested with a sample stored for 10 days at a temperature of 40±2°C and relative humidity of 75±5%.

**[Table 3]**

| Evaluation example and result | | Example 6 | Example 7 | Example 8 | Example 9 | Example 10 | Example 11 | Example 12 | Example 13 |
|---|---|---|---|---|---|---|---|---|---|
| Stabilizer | | Sorbitol 5.0 %(w/v) | Sucrose 10.0 %(w/v) | Trehalose 10.0 %(w/v) | Threonin e 300 mM | Proline 300 mM | Arginine 100 mM Leucine 50 mM | Taurine 300 mM | Glycine 300 mM |
| Antibody (mg/ml) | | 180 | 180 | 180 | 180 | 180 | 180 | 180 | 180 |
| Polysorbate 80 (%(w/v)) | | 0.02 | 0.02 | 0.02 | 0.02 | 0.02 | 0.02 | 0.02 | 0.02 |
| Sodium acetate (mM) | | 20 | 20 | 20 | 20 | 20 | 20 | 20 | 20 |
| Initi al | Turbidity | 0.0066 | 0.01 | 0.0076 | 0.0073 | 0.0074 | 0.0132 | 0.0059 | 0.0079 |
| | Main component content (Main peak%) | 99.62 | 99.66 | 99.56 | 99.72 | 99.66 | 99.71 | 99.74 | 99.7 |
| | High molecular weight of component content (%) | 0.38 | 0.34 | 0.44 | 0.28 | 0.34 | 0.29 | 0.26 | 0.3 |
| | Low molecular weight of component content (%) | 0 | 0 | 0 | 0 | 0 | 0 | 0 | 0 |
| | Viscosity (cP) | 7.48 | 7.62 | 10.51 | 6.85 | 7.31 | 5.91 | 5.44 | 6.73 |
| Ther mal acce lerat ion | Turbidity | 0.01 | 0.0037 | 0.0241 | 0.0168 | 0.0129 | 0.0046 | 0.0298 | 0.0142 |
| | Main component content (Main peak%) | 99.13 | 99.12 | 99.06 | 99.35 | 99.24 | 99.34 | 99.37 | 99.25 |
| | High molecular weight of component content (%) | 0.69 | 0.69 | 0.75 | 0.46 | 0.57 | 0.42 | 0.42 | 0.55 |
| | Low molecular weight of component content (%) | 0.18 | 0.19 | 0.19 | 0.19 | 0.19 | 0.24 | 0.2 | 0.19 |
| | Viscosity (cP) | 8.1 | 8.06 | 10.41 | 7.05 | 7.5 | 5.94 | 6.26 | 7.29 |

Under initial conditions, it was found that all of Examples 6 to 13 show turbidity of 0.03 or less, main component content of 98% or more, high molecular weight of component content of 1.5% or less, low molecular weight of component content of 1% or less, viscosity of 1 cP to 11 cP, and are pharmaceutically acceptable and stable.

Among them, Examples 9 and 12 were the highest as 99.72% and 99.74%, respectively, in the main component content and was the lowest as 0.28 and 0.26, respectively, in the high molecular weight of component content, indicating that the examples including threonine or taurine stabilizer had the highest stability.

Under the thermal acceleration condition, it was found that all of Examples 6 to 13 show turbidity of 0.06 or less, main component content of 97% or more, high molecular weight of component content of 2% or less, low molecular weight of component content of 1.5% or less, viscosity of 1 cP to 15 cP, and are pharmaceutically acceptable and stable.

Among them, it was found that Examples 9 and 12 were the highest as 99.35% and 99.37%, respectively, in the main component content, indicating that the examples including threonine or taurine stabilizer had the highest stability.

In Experimental Example 3, the stability of the examples including the threonine or taurine stabilizer was measured as being high, and in Experimental Example 4, various examples were additionally tested based on the above.

### Experimental Example 4: Stability experiments of several examples including histidine buffer

### Experimental Example 4-1. Comparison of stabilizer

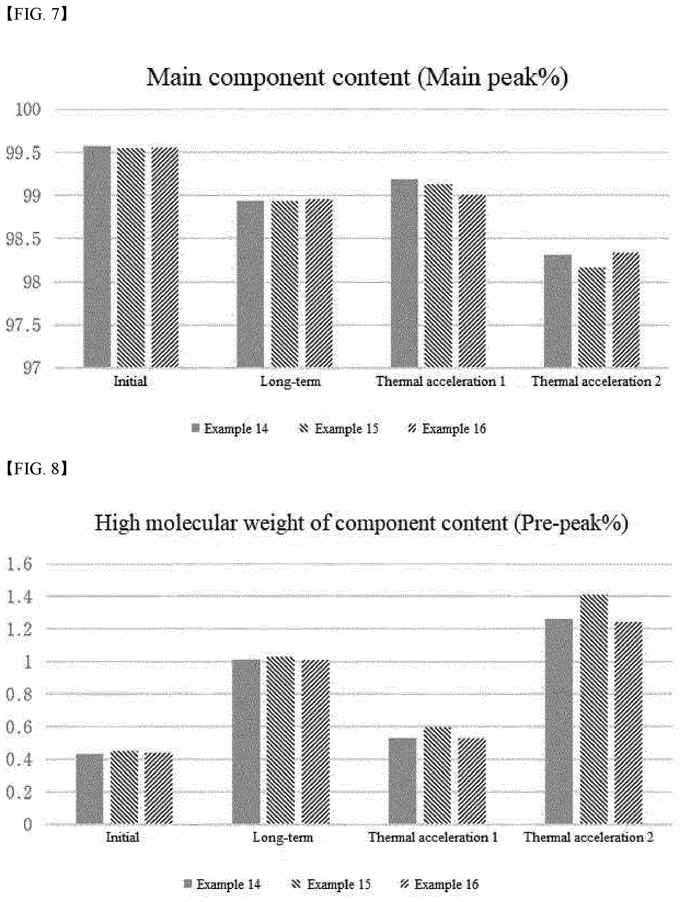

Stability of Examples 14 to 16 prepared according to Experimental Example 1 was measured under initial and long-term conditions and thermal acceleration 1 and thermal acceleration 2 conditions, and the results were shown in Table 4 and FIGS. 7 to 9 below. The initial condition was tested with a sample stored at a temperature of 5±3°C for 0 day, the long-term condition was tested with a sample stored at a temperature of 5±3°C for 4 weeks, and the thermal acceleration 1 condition was tested with a sample stored at a temperature of 40±2°C and relative humidity of 75±5% for 2 weeks, thermal acceleration 2 conditions were tested with samples stored at a temperature of 40±2°C and relative humidity of 75±5% for 4 weeks.

**[Table 4]**

| Evaluation example and result | | | Example 14 | Example 15 | Example 16 | |
|---|---|---|---|---|---|---|
| Stabilizer | | | Taurine 300 mM | Threonine 300 mM | Threonine 200 mM | |
| | | | | | Methionine 100 mM | |
| Antibody (mg/ml) | | | 180 | 180 | 180 | |
| Polysorbate 80 (%(w/v)) | | | 0.02 | 0.02 | 0.02 | |
| Histidine (mM) | | | 20 | 20 | 20 | |
| Initial | Turbidity | | 0.0163 | 0.0163 | 0.0159 | |
| | Main component content (Main peak%) | | 99.57 | 99.55 | 99.56 | |
| | High molecular weight of component content (%) | | 0.43 | 0.45 | 0.44 | |
| | Low molecular weight of component content (%) | | 0 | 0 | 0 | |
| | Number of insoluble foreign matter particle | (10 µm≤, <100 µm) | 231 | 164 | 201 | |
| | | (25 µm≤, <100 µm) | 31 | 28 | 33 | |
| | Viscosity (cP) | | | 6.56 | 7.78 | 7.35 |
| | Content of intact immunoglobulin G (Intact IgG%) | | | 97.77 | 97.49 | 98.06 |
| | Content of intact heavy chain and light chain (Intact LC+HC%) | | | 99.82 | 99.95 | 99.77 |
| | Oxidation rate (Met 106) | | | 4.1 | 4.3 | 4.4 |
| Long-term | Turbidity | | | 0.004 | 0.0062 | 0.0025 |
| | Main component content (Main peak%) | | | 98.94 | 98.94 | 98.96 |
| | High molecular weight of component content (%) | | | 1.01 | 1.03 | 1.01 |
| | Low molecular weight of component content (%) | | | 0.05 | 0.04 | 0.03 |
| | Number of insoluble foreign matter particles | | (10 µm≤, <100 µm) | 652 | 586 | 744 |
| | | | (25 µm ≤, <100 µm) | 44 | 34 | 49 |
| | Viscosity (cP) | | | 6.81 | 7.95 | 7.52 |
| | Content of intact immunoglobulin G (Intact IgG%) | | | 97.32 | 97.33 | 97.33 |
| | Content of intact heavy chain and light chain (Intact LC+HC%) | | | 99.9 | 99.84 | 99.87 |
| | Oxidation rate (Met 106) | | | 5.5 | 5.1 | 5.5 |
| Ther mal accel eratio n 1 | Turbidity | | | 0.0087 | 0.0123 | 0.0143 |
| | Main component content (Main peak%) | | | 99.19 | 99.13 | 99.01 |
| | High molecular weight of component content (%) | | | 0.53 | 0.6 | 0.53 |
| | Low molecular weight of component content (%) | | | 0.28 | 0.27 | 0.46 |
| | Number of insoluble foreign matter particles | (10 µm≤, <100 µm) | | 1799 | 890 | 1161 |
| | | (25 µm≤, <100 µm) | | 139 | 53 | 82 |
| | Viscosity (cP) | | | 6.72 | 7.92 | 7.47 |
| | Content of intact immunoglobulin G (Intact IgG%) | | | 97.15 | 97.56 | 97.5 |
| | Content of intact heavy chain and light chain (Intact LC+HC%) | | | 99.9 | 99.92 | 99.93 |
| | Oxidation rate (Met 106) | | | 4.7 | 4.6 | 5.1 |
| Ther mal accel eratio n 2 | Turbidity | | | 0.0056 | 0.0074 | 0.0071 |
| | Main component content (Main peak%) | | | 98.31 | 98.17 | 98.34 |
| | High molecular weight of component content (%) | | | 1.26 | 1.41 | 1.24 |
| | Low molecular weight of component content (%) | | | 0.43 | 0.42 | 0.43 |
| | Number of insoluble foreign matter particles | (10 µm≤, <100 µm) | | 1599 | 1256 | 670 |
| | | (25 µm≤, <100 µm) | | 166 | 48 | 7 |
| | Viscosity (cP) | | | 6.46 | 7.32 | 7.09 |
| | Content of intact immunoglobulin G (Intact IgG%) | | | 95.18 | 95.27 | 95.19 |
| | Content of intact heavy chain and light chain (Intact LC+HC%) | | | 99.52 | 99.49 | 99.48 |
| | Oxidation rate (Met 106) | | | 5.8 | 7.1 | 5.8 |

In the initial and long-term conditions, all of Examples 14 to 16 show a turbidity of 0.03 or less, a main component content of 98% or more, a high molecular weight of component content of 1.5% or less, and a low molecular weight of component content of 1% or less, and the experimental results were shown in FIGS. 7 to 9. And the number of insoluble foreign matter particles (10 µm≤, <100 µm) measured by MFI is 1,000 or less, the number of insoluble foreign matter particles (25 µm≤, <100 µm) is 150 or less, with viscosity of 1 cP to 11 cP, the content of intact immunoglobulin G of 96% or more, the content of intact heavy and light chains of 99% or more, and the oxidation rate of 6% or less, and it was found that these are pharmaceutically acceptable and stable examples.

Under the conditions of thermal acceleration 1 and 2, all of Examples 14 to 16 have a turbidity of 0.06 or less, a main component content of 97% or more, a high molecular weight of component content of 2% or less, and a low molecular weight of component content of 1.5% or less, and the experimental results are shown in FIGS. 7 to 9. And the number of insoluble foreign matter particles (10 µm≤, <100 µm) measured by MFI is 2,000 or less, the number of insoluble foreign matter particles (25 µm≤, <100 µm) is 200 or less, with viscosity of 1 cP to 15 cP, the content of intact immunoglobulin G of 93% or more, and the content of intact heavy and light chains of 98% or more, and the oxidation rate of 10% or less, and it was found that these are pharmaceutically acceptable and stable examples.

Therefore, it was found that the examples in which taurine, threonine or a mixture of threonine and methionine was added as a stabilizer and histidine was added as a buffer were pharmaceutically acceptable stable formulations.

### Experimental Example 4-2. Comparison of concentration of stabilizer

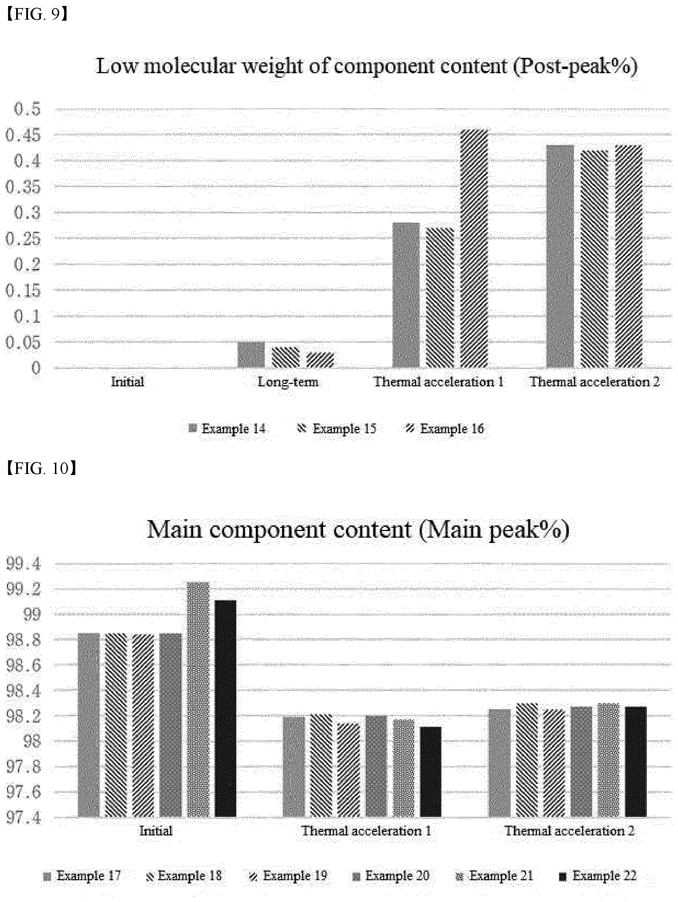

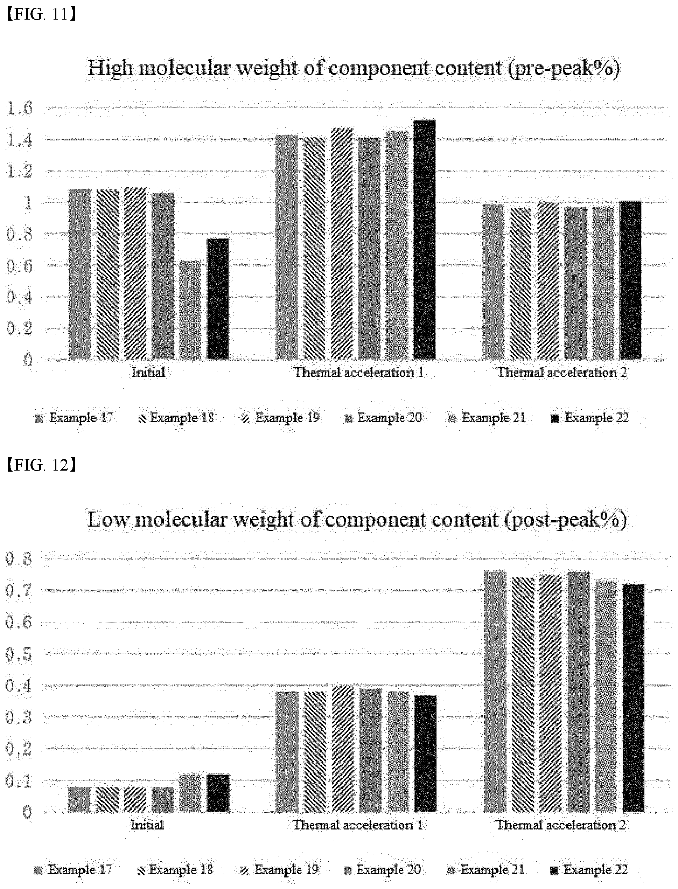

Stability of Examples 17 to 22 prepared according to Experimental Example 1 was measured under initial condition and thermal acceleration 1 and thermal acceleration 2 conditions, and the results were shown in Table 5 and FIGS. 10 to 12 below. The initial condition was tested with a sample stored at a temperature of 5±3°C for 0 day, thermal acceleration 1 condition was tested with a sample stored at a temperature of 40±2°C and relative humidity of 75±5% for 3 weeks, thermal acceleration 2 condition was tested with samples stored at a temperature of 40±2°C and relative humidity of 75±5% for 6 weeks.

**[Table 5]**

| Evaluation Example and results | | | Example 17 | Example 18 | Example 19 | Example 20 | Example 21 | Example 22 |
|---|---|---|---|---|---|---|---|---|
| Threonine(mM) | | | 140 | 180 | 160 | 160 | 110 | 189 |
| Methionine(mM) | | | 60 | 60 | 40 | 80 | 110 | 31 |
| Antibody (mg/ml) | | | 180 | 180 | 180 | 180 | 180 | 180 |
| Polysorbate 80 (%(w/v)) | | | 0.02 | 0.02 | 0.02 | 0.02 | 0.02 | 0.02 |
| Histidine (mM) | | | 10 | 10 | 10 | 10 | 10 | 10 |
| Initial | Turbidity | | 0.0074 | 0.0099 | 0.006 | 0.0056 | 0.0065 | 0.0034 |
| | Main component content (Main peak%) | | 98.85 | 98.85 | 98.84 | 98.85 | 99.25 | 99.11 |
| | High molecular weight of component content (%) | | 1.08 | 1.08 | 1.09 | 1.06 | 0.63 | 0.77 |
| | Low molecular weight of component content (%) | | 0.08 | 0.08 | 0.08 | 0.08 | 0.12 | 0.12 |
| | Number of insoluble foreign matter particles | (10 µm≤, <100 µm) | 66 | 142 | 156 | 138 | 155 | 245 |
| | | (25 µm≤, <100 µm) | 25 | 26 | 43 | 21 | 22 | 0 |
| | Content of intact immunoglobulin G (Intact IgG%) | | 99.72 | 99.74 | 99.74 | 99.74 | 99.81 | 99.79 |
| | Charge variant (Main peak%) | | 66.34 | 66.7 | 66.48 | 66.31 | 66.57 | 65.94 |
| Ther mal accel eratio n 1 | Turbidity | | 0.0046 | 0.0052 | 0.0043 | 0.011 | 0.0117 | 0.0092 |
| | Main component content (Main peak%) | | 98.19 | 98.21 | 98.14 | 98.2 | 98.17 | 98.11 |
| | High molecular weight of component content (%) | | 1.43 | 1.41 | 1.47 | 1.41 | 1.45 | 1.52 |
| | Low molecular weight of component content (%) | | 0.38 | 0.38 | 0.4 | 0.39 | 0.38 | 0.37 |
| | Number of insoluble foreign matter particles | (10 µm≤, <100 µm) | 85 | 87 | 210 | 270 | 211 | 176 |
| | | (25 µm≤, <100 µm) | 18 | 23 | 41 | 52 | 41 | 15 |
| | Content of intact immunoglobulin G (Intact IgG%) | | 95.83 | 95.76 | 95.77 | 95.43 | 96.36 | 96.63 |
| | Charge variant (Main peak%) | | 54.5 | 54.39 | 54.75 | 54.33 | 56.73 | 56.32 |
| | Turbidity | | 0.0136 | 0.011 | 0.0066 | 0.0076 | 0.0143 | 0.0256 |
| Ther mal accel eratio n2 | Intact main component content (Main peak%) | | 98.25 | 98.3 | 98.25 | 98.27 | 98.3 | 98.27 |
| | High molecular weight of component content (%) | | 0.99 | 0.96 | 1 | 0.97 | 0.97 | 1.01 |
| | Low molecular weight of component content (%) | | 0.76 | 0.74 | 0.75 | 0.76 | 0.73 | 0.72 |
| | Number of insoluble foreign matter particles | (10 µm≤, <100 µm) | 136 | 473 | 108 | 79 | 208 | 118 |
| | | (25 µm≤, <100 µm) | 11 | 70 | 21 | 16 | 10 | 12 |
| | Content of intact immunoglobulin G (Intact IgG%) | | 96.5 | 96.63 | 96.52 | 96.57 | 97.18 | 97.06 |
| | IL-6R binding affinity (%) | | 93 | 91 | 96 | 98 | 97 | 102 |
| | Charge variant (Main peak%) | | 47.78 | 47.82 | 47.48 | 47.54 | 49.22 | 49.25 |

Under the initial conditions, all of Examples 17 to 22 show a turbidity of 0.03 or less, a main component content of 98% or more, a high molecular weight of component content of 1.5% or less, a low molecular weight of component content of 1% or less, and the number of insoluble foreign matter particles (10 µm≤, <100 µm) of 1,000 or less and the number of insoluble foreign matter particles (25 µm≤ <100 µm) of 150 or less wherein the number is measured by MFI, the content of intact immunoglobulin G of 96% or more, and charge variants (main peak%) of 60% to 70%, and as a result, it was found that these are pharmaceutically acceptable and stable examples.

Under thermal acceleration 1 and 2 conditions, all of Examples 17 to 22 show a turbidity of 0.06 or less, a main component content of 97% or more, a high molecular weight of component content of 2% or less, a low molecular weight of component content of 1.5% or less, and the number of insoluble foreign matter particles (10 µm≤, <100 µm) of 2,000 or less and the number of insoluble foreign matter particles (25 µm≤, <100 µm) of 200 or less wherein the number is measured by MFI, the content of intact immunoglobulin G of 93% or more, IL-6R binding affinity of 80% to 120%, and charge variants (main peak%) of 40% to 60%, and as a result, it was found that these are pharmaceutically acceptable and stable examples.

Therefore, it was found that the example in which a mixture of threonine and methionine was added as a stabilizer and histidine was added as a buffer was a pharmaceutically acceptable stable formulation.

### Experimental Example 4-3. Comparison of concentration of buffer

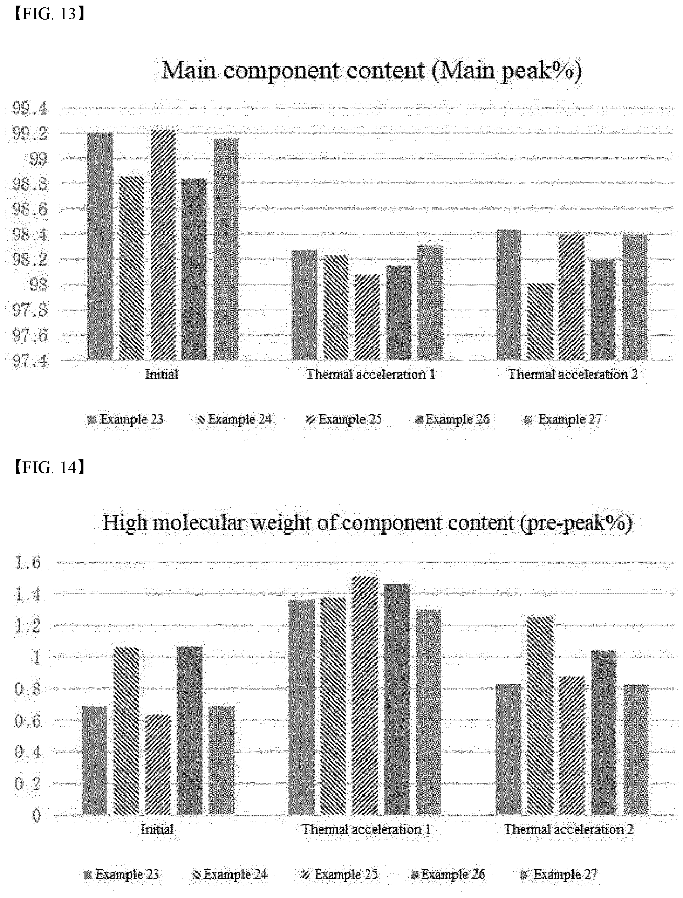

Stability of Examples 23 to 27 prepared according to Experimental Example 1 was measured under initial condition and thermal acceleration 1 and thermal acceleration 2 conditions, and the results were shown in Table 6 and FIGS. 13 to 15 below. The initial condition was tested with a sample stored at a temperature of 5±3°C for 0 day, thermal acceleration 1 condition was tested with a sample stored at a temperature of 40±2°C and relative humidity of 75±5% for 3 weeks, thermal acceleration 2 condition was tested with samples stored at a temperature of 40±2°C and relative humidity of 75±5% for 6 weeks.

**[Table 6]**

| Evaluation example and result | | | Example 23 | Example 24 | Example 25 | Example 26 | Example 27 |
|---|---|---|---|---|---|---|---|
| Histidine buffer(mM) | | | 25 | 15 | 10 | 5 | 1 |
| Antibody (mg/ml) | | | 180 | 180 | 180 | 180 | 180 |
| Polysorbate 80 (%(w/v)) | | | 0.02 | 0.02 | 0.02 | 0.02 | 0.02 |
| Threonine(mM) | | | 160 | 160 | 160 | 160 | 160 |
| Methionine(mM) | | | 60 | 60 | 60 | 60 | 60 |
| Initial | Turbidity | | 0.0055 | 0.0054 | 0.0067 | 0.0051 | 0.0086 |
| | Main component content (Main peak%) | | 99.2 | 98.86 | 99.23 | 98.84 | 99.16 |
| | High molecular weight of component content (%) | | 0.69 | 1.06 | 0.64 | 1.07 | 0.69 |
| | Low molecular weight of component content (%) | | 0.11 | 0.08 | 0.13 | 0.09 | 0.14 |
| | Number of insoluble foreign matter particle | (10 µm≤, <100 µm) | 103 | 130 | 225 | 64 | 69 |
| | | (25 µm≤, <100 µm) | 30 | 30 | 42 | 12 | 25 |
| | Content of intact immunoglobulin G (Intact IgG%) | | 99.8 | 99.73 | 99.8 | 99.74 | 99.83 |
| | Charge variant (Main peak%) | | 66.2 | 65.92 | 66 | 66.03 | 68.5 |
| Therm al accele ration 1 | Turbidity | | 0.0055 | 0.0058 | 0.0038 | 0.0035 | 0.0294 |
| | Main component content (Main peak%) | | 98.27 | 98.23 | 98.08 | 98.15 | 98.31 |
| | High molecular weight of component content (%) | | 1.36 | 1.38 | 1.51 | 1.46 | 1.3 |
| | Low molecular weight of component content (%) | | 0.37 | 0.39 | 0.42 | 0.39 | 0.39 |
| | Number of insoluble foreign matter particles | (10 µm≤, <100 µm) | 64 | 251 | 156 | 159 | 179 |
| | | (25 µm≤, <100 µm) | 5 | 31 | 23 | 34 | 37 |
| | Content of intact immunoglobulin G (Intact IgG%) | | 96.56 | 95.52 | 96.81 | 95.5 | 94.81 |
| | Charge variant (Main peak%) | | 55.87 | 54.29 | 54.4 | 54.7 | 58.67 |
| Therm al accele ration 2 | Turbidity | | 0.0326 | 0.0032 | 0.0289 | 0.0022 | 0.0457 |
| | Main component content (Main peak%) | | 98.43 | 98.01 | 98.4 | 98.2 | 98.4 |
| | High molecular weight of component content (%) | | 0.83 | 1.25 | 0.88 | 1.04 | 0.83 |
| | Low molecular weight of component content (%) | | 0.74 | 0.73 | 0.72 | 0.75 | 0.77 |
| | Number of insoluble foreign matter particles | (10 µm≤, <100 µm) | 356 | 92 | 213 | 536 | 316 |
| | | (25 µm≤, <100 µm) | 46 | 46 | 17 | 77 | 52 |
| | Content of intact immunoglobulin G (Intact IgG%) | | 97.41 | 97.45 | 97.44 | 96.41 | 97.09 |
| | IL-6R binding affinity | | 99 | 101 | 96 | 100 | 99 |
| | Charge variant (Main peak%) | | 49.34 | 48.23 | 47.15 | 48.23 | 51.21 |

Under the initial conditions, all of Examples 23 to 27 show a turbidity of 0.03 or less, a main component content of 98% or more, a high molecular weight of component content of 1.5% or less, a low molecular weight of component content of 1% or less, and the number of insoluble foreign matter particles (10 µm≤, <100 µm) of 1,000 or less and the number of insoluble foreign matter particles (25 µm≤ <100 µm) of 150 or less wherein the number is measured by MFI, the content of intact immunoglobulin G of 96% or more, and charge variants (main peak%) of 60% to 70%, and as a result, it was found that these are pharmaceutically acceptable and stable examples.

Under thermal acceleration 1 and 2 conditions, all of Examples 23 to 27 show a turbidity of 0.06 or less, a main component content of 97% or more, a high molecular weight of component content of 2% or less, a low molecular weight of component content of 1.5% or less, and the number of insoluble foreign matter particles (10 µm≤, <100 µm) of 2,000 or less and the number of insoluble foreign matter particles (25 µm≤, <100 µm) of 200 or less wherein the number is measured by MFI, the content of intact immunoglobulin G of 93% or more, IL-6R binding affinity of 80% to 120%, and charge variants (main peak%) of 40% to 60%, and as a result, it was found that these are pharmaceutically acceptable and stable examples.

### Experimental Example 4-4. Comparison of concentration of antibody

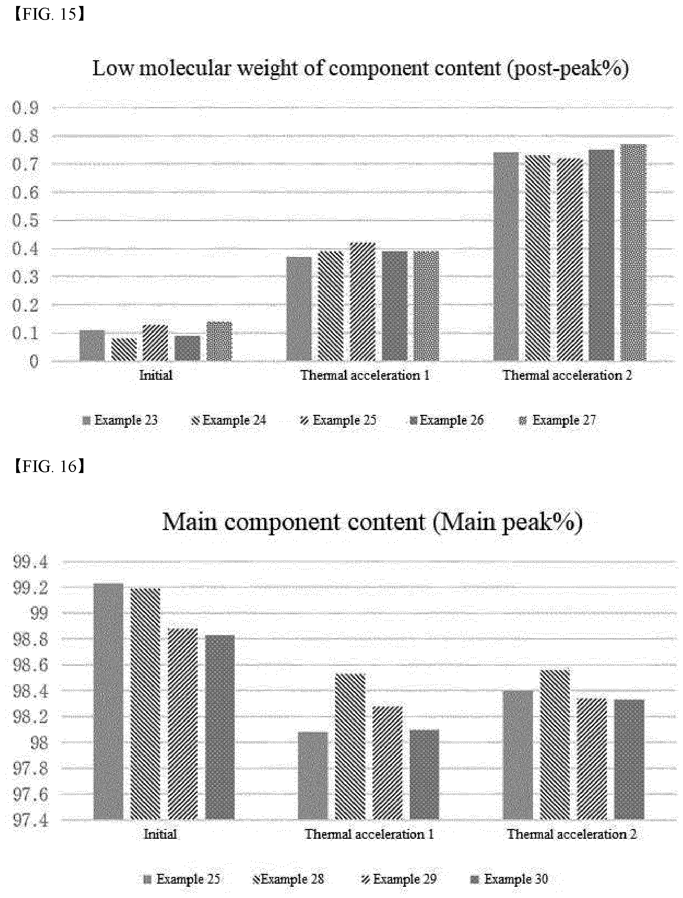

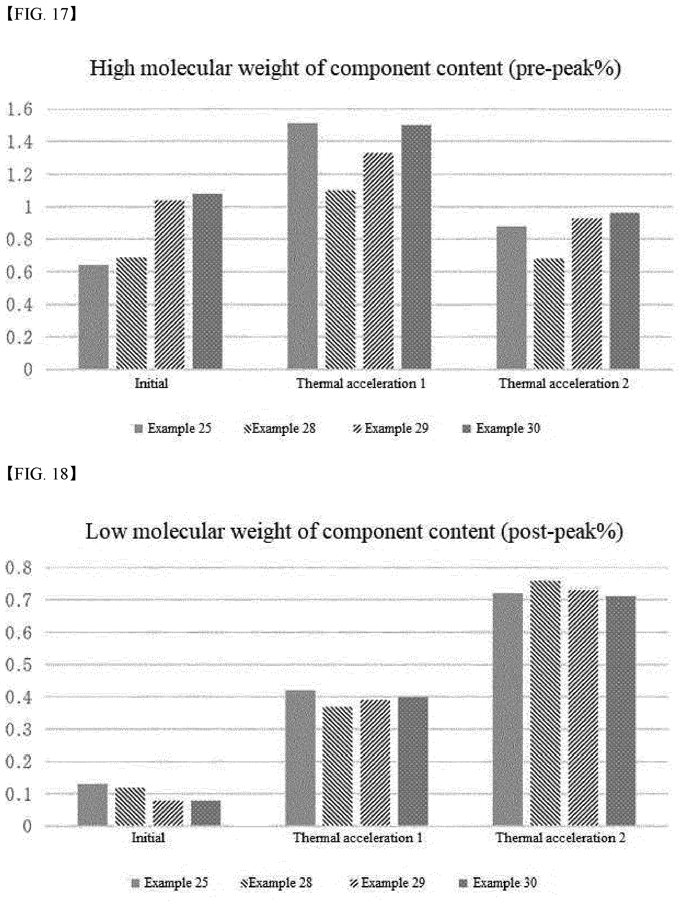

Stability of Examples 25 and 28 to 30 prepared according to Experimental Example 1 was measured under initial condition and thermal acceleration 1 and thermal acceleration 2 conditions, and the results were shown in Table 7 and FIGS. 16 to 18 below. The initial condition was tested with a sample stored at a temperature of 5±3°C for 0 day, thermal acceleration 1 condition was tested with a sample stored at a temperature of 40±2°C and relative humidity of 75±5% for 3 weeks, thermal acceleration 2 condition was tested with samples stored at a temperature of 40±2°C and relative humidity of 75±5% for 6 weeks.

**[Table 7]**

| Evaluation example and result | | | Example 25 | Example 28 | Example 29 | Example 30 |
|---|---|---|---|---|---|---|
| Antibody(mg/ml) | | | 180 | 100 | 160 | 200 |
| Polysorbate 80 (%(w/v)) | | | 0.02 | 0.02 | 0.02 | 0.02 |
| Threonine(mM) | | | 160 | 160 | 160 | 160 |
| Methionine(mM) | | | 60 | 60 | 60 | 60 |
| Histidine (mM) | | | 10 | 10 | 10 | 10 |
| Initial | Turbidity | | 0.0067 | 0.0056 | 0.005 | 0.0082 |
| | Main component content (Main peak%) | | 99.23 | 99.19 | 98.88 | 98.83 |
| | High molecular weight of component content (%) | | 0.64 | 0.69 | 1.04 | 1.08 |
| | Low molecular weight of component content (%) | | 0.13 | 0.12 | 0.08 | 0.08 |
| | Number of insoluble foreign matter particles | (10 µm≤, <100 µm) | 225 | 210 | 158 | 120 |
| | | (25 µm≤, <100 µm) | 42 | 41 | 52 | 12 |
| | Content of intact immunoglobulin G (Intact IgG%) | | 99.8 | 99.75 | 99.7 | 99.74 |
| | Charge variant (Main peak%) | | 66 | 66.24 | 66.22 | 66.17 |
| Therm al accele ration 1 | Turbidity | | 0.0038 | 0.008 | 0.0036 | 0.0056 |
| | Main component content (Main peak%) | | 98.08 | 98.53 | 98.28 | 98.1 |
| | High molecular weight of component content (%) | | 1.51 | 1.1 | 1.33 | 1.5 |
| | Low molecular weight of component content (%) | | 0.42 | 0.37 | 0.39 | 0.4 |
| | Number of insoluble foreign matter particles | (10 µm≤, <100 µm) | 156 | 143 | 126 | 128 |
| | | (25 µm≤, <100 µm) | 23 | 14 | 54 | 29 |
| | Content of intact immunoglobulin G (Intact IgG%) | | 96.81 | 96.59 | 95.91 | 95.78 |
| | Charge variant (Main peak%) | | 54.4 | 55.69 | 54.47 | 54.2 |
| Therm al accele ration 2 | Turbidity | | 0.0289 | 0.0081 | 0.0056 | 0.0035 |
| | Main component content (Main peak%) | | 98.4 | 98.56 | 98.34 | 98.33 |
| | High molecular weight of component content (%) | | 0.88 | 0.68 | 0.93 | 0.96 |
| | Low molecular weight of component content (%) | | 0.72 | 0.76 | 0.73 | 0.71 |
| | Number of insoluble foreign matter particles | (10 µm≤, <100 µm) | 213 | 681 | 508 | 635 |
| | | (25 µm≤, <100 µm) | 17 | 89 | 69 | 34 |
| | Content of intact immunoglobulin G (Intact IgG%) | | 97.44 | 96.81 | 96.54 | 96.44 |
| | IL-6R binding affinity (%) | | 96 | 83 | 101 | 96 |
| | Charge variant (Main peak%) | | 47.15 | 47.5 | 47.77 | 47.68 |

Under the initial conditions, all of Examples 25 and 28 to 30 show a turbidity of 0.03 or less, a main component content of 98% or more, a high molecular weight of component content of 1.5% or less, a low molecular weight of component content of 1% or less, and the number of insoluble foreign matter particles (10 µm≤, <100 µm) of 1,000 or less and the number of insoluble foreign matter particles (25 µm≤, <100 µm) of 150 or less wherein the number is measured by MFI, the content of intact immunoglobulin G of 96% or more, and charge variants (main peak%) of 60% to 70%, and as a result, it was found that these are pharmaceutically acceptable and stable examples.

Under thermal acceleration 1 and 2 conditions, all of Examples 25, 28 to 30 show a turbidity of 0.06 or less, a main component content of 97% or more, a high molecular weight of component content of 2% or less, a low molecular weight of component content of 1.5% or less, and the number of insoluble foreign matter particles (10 µm≤, <100 µm) of 2,000 or less and the number of insoluble foreign matter particles (25 µm≤, <100 µm) of 200 or less wherein the number is measured by MFI, the content of intact immunoglobulin G of 93% or more, IL-6R binding affinity of 80% to 120%, and charge variants (main peak%) of 40% to 60%, and as a result, it was found that these are pharmaceutically acceptable and stable examples.

### Experimental Example 4-5. Comparison of pH

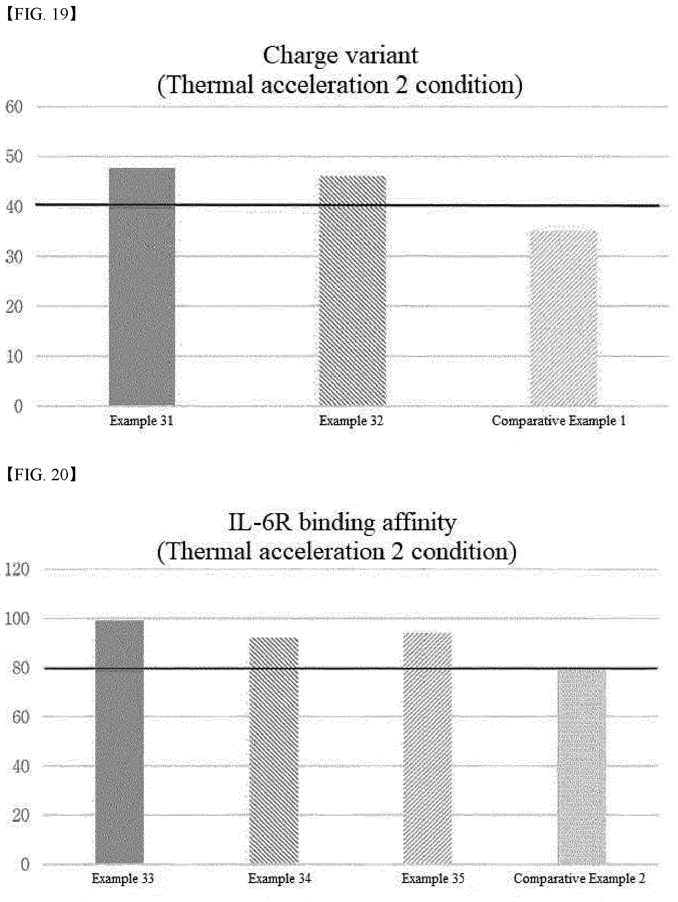

Stability of Examples 31 and 32 and Comparative Example 1 prepared according to Experimental Example 1 was measured under initial condition and thermal acceleration 1 and thermal acceleration 2 conditions, and the results were shown in Table 8 and FIG. 19 below. The initial condition was tested with a sample stored at a temperature of 5±3°C for 0 day, thermal acceleration 1 condition was tested with a sample stored at a temperature of 40±2°C and relative humidity of 75±5% for 3 weeks, thermal acceleration 2 condition was tested with samples stored at a temperature of 40±2°C and relative humidity of 75±5% for 6 weeks.

**[Table 8]**

| Evaluation example and result | | | Example 31 | Example 32 | Comparative Example 1 |
|---|---|---|---|---|---|
| pH | | | 5.7 | 6.3 | 7 |
| Antibody (mg/ml) | | | 180 | 180 | 180 |
| Polysorbate 80 (%(w/v)) | | | 0.02 | 0.02 | 0.02 |
| Threonine(mM) | | | 160 | 160 | 160 |
| Methionine(mM) | | | 60 | 60 | 60 |
| Histidine (mM) | | | 10 | 10 | 10 |
| Initial | Turbidity | | 0.005 | 0.0033 | 0.005 |
| | Main component content (Main peak%) | | 98.89 | 98.81 | 99.17 |
| | High molecular weight of component content (%) | | 1.03 | 1.12 | 0.69 |
| | Low molecular weight of component content (%) | | 0.08 | 0.08 | 0.13 |
| | Number of insoluble foreign matter particles | (10 µm≤, <100 µm) | 67 | 446 | 94 |
| | | (25 µm≤, <100 µm) | 10 | 101 | 4 |
| | Content of intact immunoglobulin G (Intact IgG%) | | 99.73 | 99.72 | 99.83 |
| | Charge variant (Main peak%) | | 66.26 | 65.9 | 68.09 |
| Therm al accele | Turbidity | | 0.0033 | 0.004 | 0.0064 |
| | Main component content (Main peak%) | | 98.24 | 98.13 | 97.81 |
| | High molecular weight of component content (%) | | 1.34 | 1.5 | 1.66 |
| ration 1 | Low molecular weight of component content (%) | | 0.42 | 0.38 | 0.53 |
| | Number of insoluble foreign matter particles | (10 µm≤, <100 µm) | 120 | 203 | 179 |
| | | (25 µm≤, <100 µm) | 21 | 43 | 28 |
| | Content of intact immunoglobulin G (Intact IgG%) | | 95.67 | 95.64 | 95.14 |
| | Charge variant (Main peak%) | | 53.19 | 54.62 | 47.1 |
| Therm al accele ration 2 | Turbidity | | 0.0043 | 0.0077 | 0.0118 |
| | Main component content (Main peak%) | | 98.3 | 98.19 | 97.53 |
| | High molecular weight of component content (%) | | 0.89 | 1.07 | 1.39 |
| | Low molecular weight of component content (%) | | 0.81 | 0.74 | 1.07 |
| | Number of insoluble foreign matter particles | (10 µm≤, <100 µm) | 321 | 146 | 108 |
| | | (25 µm≤, <100 µm) | 52 | 25 | 19 |
| | Content of intact immunoglobulin G (Intact IgG%) | | 96.33 | 96.44 | 95.19 |
| | IL-6R binding affinity (%) | | 103 | 99 | 82 |
| | Charge variant (Main peak%) | | 47.69 | 46.06 | 34.89 |

Under the initial conditions, all of Examples 31 and 32 show a turbidity of 0.03 or less, a main component content of 98% or more, a high molecular weight of component content of 1.5% or less, a low molecular weight of component content of 1% or less, and the number of insoluble foreign matter particles (10 µm≤, <100 µm) of 1,000 or less and the number of insoluble foreign matter particles (25 µm≤, <100 µm) of 150 or less wherein the number is measured by MFI, the content of intact immunoglobulin G of 96% or more, and charge variants (main peak%) of 60% to 70%, and as a result, it was found that these are pharmaceutically acceptable and stable examples.

Under thermal acceleration 1 and 2 conditions, all of Examples 31 and 32 show a turbidity of 0.06 or less, a main component content of 97% or more, a high molecular weight of component content of 2% or less, a low molecular weight of component content of 1.5% or less, and the number of insoluble foreign matter particles (10 µm≤, <100 µm) of 2,000 or less and the number of insoluble foreign matter particles (25 µm≤, <100 µm) of 200 or less wherein the number is measured by MFI, the content of intact immunoglobulin G of 93% or more, IL-6R binding affinity of 80% to 120%, and charge variants (main peak%) of 40% to 60%, and as a result, it was found that these are pharmaceutically acceptable and stable examples.

However, in Comparative Example 1, it was confirmed that the charge variant (main peak%) was less than 40% under thermal acceleration 2 condition, and through this, it was found that the stability of the example having a pH of 7 or higher was deteriorated.

### Experimental Example 4-6. Comparison of surfactant

Stability of Examples 33 to 35 and Comparative Example 2 prepared according to Experimental Example 1 was measured under initial condition and thermal acceleration 1 and thermal acceleration 2 conditions, and the results were shown in Table 9 and FIGS. 20 and 21 below. The initial condition was tested with a sample stored at a temperature of 5±3°C for 0 day, thermal acceleration 1 condition was tested with a sample stored at a temperature of 40±2°C and relative humidity of 75±5% for 3 weeks, thermal acceleration 2 condition was tested with samples stored at a temperature of 40±2°C and relative humidity of 75±5% for 6 weeks.

**[Table 9]**

| Evaluation formulation and result | | | Example 33 | Example 34 | Example 35 | Comparative Example 2 |
|---|---|---|---|---|---|---|
| Surfactant | | | Polysorb ate 80 0.01 % (w/v) | Polysorba te 80 0.05 % (w/v) | Poloxame r 188 0.02% (w/v) | Polysorbate 80 0.00 % (w/v) |
| Antibody (mg/ml) | | | 180 | 180 | 180 | 180 |
| Threonine(mM) | | | 160 | 160 | 160 | 160 |
| Methionine(mM) | | | 60 | 60 | 60 | 60 |
| Histidine (mM) | | | 10 | 10 | 10 | 10 |
| Initial | Turbidity | | 0.0153 | 0.0093 | 0.0073 | 0.0048 |
| | Main component content (Main peak%) | | 98.86 | 98.84 | 99.1 | 99.16 |
| | High molecular weight of component content (%) | | 1.07 | 1.07 | 0.78 | 0.73 |
| | Low molecular weight of component content (%) | | 0.06 | 0.08 | 0.12 | 0.12 |
| | Number of insoluble foreign matter particles | (10 µm≤, <100 µm) | 123 | 58 | 805 | 110 |
| | | (25 µm≤, <100 µm) | 65 | 10 | 100 | 18 |
| | Content of intact immunoglobulin G (Intact IgG%) | | 99.76 | 99.78 | 99.81 | 99.83 |
| | Charge variant (Main peak%) | | 66.18 | 66.15 | 65.72 | 68.84 |
| Therm al accele ration 1 | Turbidity | | 0.0023 | 0.0055 | 0.0029 | 0.0038 |
| | Main component content (Main peak%) | | 98.18 | 98.15 | 98.2 | 98.36 |
| | High molecular weight of component content (%) | | 1.44 | 1.45 | 1.44 | 1.27 |
| | Low molecular weight of component content (%) | | 0.38 | 0.39 | 0.36 | 0.37 |
| | Number of insoluble foreign matter particles | (10 µm≤, <100 µm) | 650 | 593 | 64 | 124 |
| | | (25 µm≤, <100 µm) | 77 | 26 | 23 | 46 |
| | Content of intact immunoglobulin G (Intact IgG%) | | 96 | 95.98 | 96.62 | 95.05 |
| | Charge variant (Main peak%) | | 54.56 | 54.4 | 55.68 | 58.24 |
| Therm al accele ration 2 | Turbidity | | 0.005 | 0.0031 | 0.0072 | 0.0098 |
| | Main component content (Main peak%) | | 98.3 | 98.27 | 98.32 | 98.48 |
| | High molecular weight of component content (%) | | 0.98 | 0.99 | 0.94 | 0.82 |
| | Low molecular weight of component content (%) | | 0.73 | 0.74 | 0.74 | 0.7 |
| | Number of insoluble foreign matter particles | (10 µm≤, <100 µm) | 861 | 657 | 61 | 434 |
| | | (25 µm≤, <100 µm) | 129 | 102 | 15 | 121 |
| | Content of intact immunoglobulin G (Intact IgG%) | | 96.52 | 96.66 | 96.74 | 96.88 |
| | IL-6R binding affinity (%) | | 99 | 92 | 94 | 79 |
| | Charge variant (Main peak%) | | 47.08 | 47.53 | 48.07 | 50.68 |

Under the initial conditions, all of Examples 33 to 35 show a turbidity of 0.03 or less, a main component content of 98% or more, a high molecular weight of component content of 1.5% or less, a low molecular weight of component content of 1% or less, and the number of insoluble foreign matter particles (10 µm≤, <100 µm) of 1,000 or less and the number of insoluble foreign matter particles (25 µm≤, <100 µm) of 150 or less wherein the number is measured by MFI, the content of intact immunoglobulin G of 96% or more, and charge variants (main peak%) of 60% to 70%, and as a result, it was found that these are pharmaceutically acceptable and stable examples.

Under thermal acceleration 1 and 2 conditions, all of Examples 33 to 35 show a turbidity of 0.06 or less, a main component content of 97% or more, a high molecular weight of component content of 2% or less, a low molecular weight of component content of 1.5% or less, and the number of insoluble foreign matter particles (10 µm≤, <100 µm) of 2,000 or less and the number of insoluble foreign matter particles (25 µm≤, <100 µm) of 200 or less wherein the number is measured by MFI, the content of intact immunoglobulin G of 93% or more, IL-6R binding affinity of 80% to 120%, and charge variants (main peak%) of 40% to 60%, and as a result, it was found that these are pharmaceutically acceptable and stable examples.

On the other hand, in Comparative Example 2, the IL-6R binding affinity was less than 80% under the thermal acceleration 2 condition. Through this, it was found that the stability of the example in which the surfactant was not included was inferior.

## Claims

1. A stable pharmaceutical formulation, comprising:
(A) an antibody or antigen-binding fragment thereof that binds to an interleukin-6 receptor;
(B) a surfactant;
(C) a stabilizer which is i) an amino acid or an amino acid derivative, ii) a sugar or a sugar alcohol or a mixture thereof; and
(D) a buffer,
wherein the antibody or antigen-binding fragment thereof comprises a light chain variable region comprising a CDR1 domain comprising the amino acid sequence of SEQ ID NO: 1, a CDR2 domain comprising the amino acid sequence of SEQ ID NO: 2, and CDR3 domain comprising amino acids of SEQ ID NO: 3; and a heavy chain variable region comprising a CDR1 domain comprising the amino acid sequence of SEQ ID NO: 4, a CDR2 domain comprising the amino acid sequence of SEQ ID NO: 5, and a CDR3 domain comprising the amino acid sequence of SEQ ID NO: 6.

2. The stable pharmaceutical formulation of claim 1, wherein the antibody or antigen-binding fragment thereof comprises a light chain variable region comprising the amino acid sequence of SEQ ID NO: 7; and a heavy chain variable region comprising the amino acid sequence of SEQ ID NO: 8.

3. The stable pharmaceutical formulation of claim 1, wherein the antibody or antigen-binding fragment thereof comprises a light chain comprising the amino acid sequence of SEQ ID NO: 9; and a heavy chain comprising the amino acid sequence of SEQ ID NO: 10.

4. The stable pharmaceutical formulation of claim 1, wherein the antibody or antigen-binding fragment thereof is Tocilizumab.

5. The stable pharmaceutical formulation of claim 1, wherein the pharmaceutical formulation is liquid.

6. The stable pharmaceutical formulation of claim 1, wherein the pharmaceutical formulation has an effect for treating diseases associated with interleukin-6 receptor.

7. The stable pharmaceutical formulation of claim 6, wherein the diseases associated with the interleukin-6 receptor are any one or more selected from the group consisting of rheumatoid arthritis, adult onset still's disease, systemic juvenile idiopathic arthritis, polyarticular juvenile idiopathic arthritis, castleman's disease, giant cell arteritis, Takayasu's arteritis, systemic sclerosis, systemic sclerosis-associated interstitial lung disease, cytokine release syndrome, hand osteoarthritis, polymyalgia rheumatica, antineutrophil cytoplasmic antibody associated vasculitis, relapsing polychondritis, type 2 diabetes, ankylosing spondylitis, axial spondyloarthritis, psoriasis, psoriatic arthritis, inflammatory bowel disease, Crohn's disease, ulcerative colitis, thyroid associated ophthalmopathy, rheumatoid arthritis-associated cardiovascular disease, acute graft versus host disease, non-ST-segment elevation MI, systemic lupus erythematosus, schizophrenia, uveitis, ovarian cancer, neuromyelitis optica, glomerulonephritis, chronic glomerulonephritis, colorectal cancer, pneumonia, and lung cancer.

8. The stable pharmaceutical formulation of claim 1, wherein the concentration of antibody or antigen-binding fragment thereof is 1 to 300 mg/ml.

9. The stable pharmaceutical formulation of claim 1, wherein the surfactant is polysorbate, poloxamer, or a mixture thereof.

10. The stable pharmaceutical formulation of claim 9, wherein the surfactant is polysorbate 20, polysorbate 40, polysorbate 60, polysorbate 80, or a mixture thereof.

11. The stable pharmaceutical formulation of claim 10, wherein the surfactant is polysorbate 80.

12. The stable pharmaceutical formulation of claim 1, wherein the concentration of the surfactant is 0.001 to 1% (w/v).

13. The stable pharmaceutical formulation of claim 1, wherein the amino acid or amino acid derivative is any one or more selected from the group consisting of threonine, methionine, arginine, proline, leucine, glycine, and taurine.

14. The stable pharmaceutical formulation of claim 1, wherein the sugar or sugar alcohol is any one or more selected from the group consisting of sucrose, trehalose, and sorbitol.

15. The stable pharmaceutical formulation of claim 1, wherein the concentration of the amino acid or amino acid derivative is 10 to 500 mM.

16. The stable pharmaceutical formulation of claim 1, wherein the concentration of the sugar or sugar alcohol is 0.1 to 30% (w/v).

17. The stable pharmaceutical formulation of claim 1, wherein the stabilizer is a mixture of threonine and methionine.

18. The stable pharmaceutical formulation of claim 17, wherein a concentration ratio of the mixture of threonine and methionine is 1:1 to 10:1.

19. The stable pharmaceutical formulation of claim 17, wherein a concentration of the threonine is 5 to 300 mM, and
a concentration of the methionine is 5 to 200 mM.

20. The stable pharmaceutical formulation of claim 1, wherein the buffer is histidine or a salt thereof, acetic acid or a salt thereof, phosphoric acid or a salt thereof, citric acid or a salt thereof, succinic acid or a salt thereof, or a mixture thereof.

21. The stable pharmaceutical formulation of claim 1, wherein a content of the buffer is 0.1 to 50 mM.

22. The stable pharmaceutical formulation of claim 1, wherein the pharmaceutical formulation has a pH of 5 or more and less than 7.

23. A stable pharmaceutical formulation, comprising:
(A) 1 to 300 mg/ml of an antibody or antigen-binding fragment thereof comprising a light chain variable region comprising a CDR1 domain comprising the amino acid sequence of SEQ ID NO: 1, a CDR2 domain comprising the amino acid sequence of SEQ ID NO: 2, and a CDR3 domain comprising the amino acid sequence of SEQ ID NO: 3; and a heavy chain variable region comprising a CDR1 domain comprising the amino acid sequence of SEQ ID NO: 4, a CDR2 domain comprising the amino acid sequence of SEQ ID NO: 5, and a CDR3 domain comprising the amino acid sequence of SEQ ID NO: 6;
(B) 0.001 to 1% (w/v) of a surfactant;
(C) 10 to 500 mM of an amino acid or an amino acid derivative as a stabilizer; and
(D) 0.1 to 50 mM of a buffer.

24. A stable pharmaceutical formulation, comprising:
(A) 1 to 300 mg/ml of an antibody or antigen-binding fragment thereof comprising a light chain variable region comprising a CDR1 domain comprising the amino acid sequence of SEQ ID NO: 1, a CDR2 domain comprising the amino acid sequence of SEQ ID NO: 2, and a CDR3 domain comprising the amino acid sequence of SEQ ID NO: 3; and a heavy chain variable region comprising a CDR1 domain comprising the amino acid sequence of SEQ ID NO: 4, a CDR2 domain comprising the amino acid sequence of SEQ ID NO: 5, and a CDR3 domain comprising the amino acid sequence of SEQ ID NO: 6;
(B) 0.001 to 1% (w/v) of a surfactant;
(C) 0.1 to 30% (w/v) of a sugar or sugar alcohol as a stabilizer; and
(D) 0.1 to 50 mM of a buffer.

25. The stable pharmaceutical formulation of any one of claims 1 to 24, wherein the pharmaceutical formulation is for intravenous or subcutaneous administration.

26. The stable pharmaceutical formulation of claim 25, wherein the pharmaceutical formulation for subcutaneous administration is used as a formulation for intravenous administration through a dilution step.

27. A vial filled with the stable pharmaceutical formulation set forth in any one of claims 1 to 26.

28. A cartridge filled with the stable pharmaceutical formulation set forth in any one of claims 1 to 26.

29. A pre-filled syringe filled with the stable pharmaceutical formulation set forth in any one of claims 1 to 26.

30. An auto-injector comprising the pre-filled syringe set forth in any one of claims 1 to 26.
